# EUROPEAN PATENT SPECIFICATION

(11) **EP 4 324 811 B1**
(45) Date of publication and mention of the grant of the patent: **10.12.2025**
(21) Application number: 22787430.2
(22) Date of filing: 07.04.2022
(51) Int. Cl.: C07C 17/25, C07C 17/23, C07C 21/18, C07C 19/10, B01J 21/18, B01J 23/26, B01J 23/44, B01J 27/12, B01J 27/125, B01J 37/02, B01J 37/04, C07C 17/278, B01J 23/42, B01J 37/00

(54) **METHOD FOR PREPARING 2,3,3,3-TETRAFLUOROPROPENE**
VERFAHREN ZUR HERSTELLUNG VON 2,3,3,3-TETRAFLUORPROPEN
PROCÉDÉ DE PRÉPARATION DE 2,3,3,3-TÉTRAFLUOROPROPÈNE

(30) Priority: 15.04.2021 CN 202110404614; 15.04.2021 CN 202110404622; 15.04.2021 CN 202110404625
(43) Date of publication of application: 21.02.2024
(73) Proprietor: Zhejiang Research Institute of Chemical Industry Co., Ltd., Hangzhou, Zhejiang 310023 (CN); Sinochem Lantian Co., Ltd., Hangzhou, Zhejiang 310051 (CN)
(72) Inventor: LIU, Minyang, Hangzhou, Zhejiang 310023 (CN); YU, Wanjin, Hangzhou, Zhejiang 310023 (CN); LIN, Shengda, Hangzhou, Zhejiang 310023 (CN); XIAO, Xinbao, Hangzhou, Zhejiang 310023 (CN); LUO, Xia, Hangzhou, Zhejiang 310023 (CN); LIU, Wucan, Hangzhou, Zhejiang 310023 (CN); ZHANG, Jianjun, Hangzhou, Zhejiang 310023 (CN)
(74) Representative: Glawe, Delfs, Moll
(86) International application number: PCT/CN2022/085539
(87) International publication number: WO 2022/218204

(56) References cited:
- WO-A1-2019/003896
- CN-A- 101 535 224
- CN-A- 109 438 170
- CN-A- 110 776 394
- CN-A- 113 527 040
- CN-A- 113 527 045
- US-A1- 2010 197 981

## Description

### Technical Field

The present disclosure relates to preparation of 2,3,3,3-tetrafluoropropene, in particular to a method for preparing 2,3,3,3-tetrafluoropropene by reactions of telomerization and removal (dehydrochlorination, dehydrofluorination, dehydrogenation and the like) in two steps with trifluoroethylene as a raw material.

### Background

2,3,3,3-tetrafluoropropene has an ozone depression potential (ODP) value of zero, a global warming potential (GWP) value of less than 1, lower life cycle climate performance (LCCP) than a traditional refrigerant HFC-134a, better system refrigeration performance than the HFC-134a and same atmospheric decomposition products as the HFC-134a. Thus, the 2,3,3,3-tetrafluoropropene is considered as the most promising substitute for automotive refrigerants at present, and has been accepted by many major automobile manufacturers. At present, the 2,3,3,3-tetrafluoropropene has the following preparation routes.

### 1. Hexafluoropropene route:

The 2,3,3,3-tetrafluoropropene is prepared by reactions in four steps with hexafluoropropene as a raw material: (1) subjecting hexafluoropropene and hydrogen to a hydrogenation reaction to prepare 1,1,1,2,3,3-hexafluoropropane (HFC-236ea); (2) subjecting the HFC-236ea to a dehydrofluorination reaction under the action of a catalyst to prepare 1,1,1,2,3-pentafluoropropene (HFO-1225ye); (3) subjecting the HFO-1225ye and hydrogen to a hydrogenation reaction to prepare 1,1,1,2,3-pentafluoropropane (HFC-245eb); and (4) subjecting the HFC-245eb to a dehydrofluorination reaction under the action of a catalyst to prepare the 2,3,3,3-tetrafluoropropene.

In a United States patent US20070179324A and Chinese patents CN101544536A, CN102267869A, CN102026947A and the like, methods for preparing 2,3,3,3-tetrafluoropropene by reactions of hydrogenation, dehydrofluorination, re-hydrogenation and re-dehydrofluorination in four steps with hexafluoropropene as a raw material are disclosed. These methods have the characteristics of a simple process, a mature technology and the like, but have the problems of many reaction steps, a variety of intermediate products requiring separation and purification, complicated process steps, large investment in equipment, low reaction yield, high separation cost, high energy consumption and the like.

In order to overcome the shortcomings of technologies in the above patents, a Chinese patent CN103449963B discloses a method for synthesizing 2,3,3,3-tetrafluoropropene by continuous reactions in multiple steps with hexafluoropropene as a raw material. Continuous production based on direct reactions of intermediate products, such as the HFC-236ea, the HFO-1225ye and the HFC-245eb, without separation can be realized. However, without separation and purification of the intermediate products, it is indicated that impurities will be accumulated and increased in reaction materials constantly, the yield of the target product 2,3,3,3-tetrafluoropropene is eventually affected, and meanwhile, difficulty in rectification and separation of a 2,3,3,3-tetrafluoropropene product is increased.

### 2. Tetrachloropropene (TCP) route:

A patent CN101395108B discloses a method for preparing 2,3,3,3-tetrachloropropene by reactions in three steps with 1,1,2,3-tetrachloropropene as a raw material. The method includes the following reaction steps: (1) subjecting 1,1,2,3-tetrachloropropene and hydrogen fluoride (HF) to a gas phase fluorination reaction to prepare 2-chloro-3,3,3-trifluoropropene (HCFO-1233xf) with a selectivity of 80-96%, wherein when Cr₂O₃ and FeCl₃/AC catalysts are used, the selectivity reaches 96%, and the conversion rate is only 20%; (2) subjecting the HCFO-1233xF and HF to an addition reaction with SbCl₅ as a catalyst to produce 2-chloro-1,1,1,2-tetrafluoropropane (HCFC-244bb); and (3) subjecting the HCFC-244bb to a gas phase dehydrochlorination reaction under the action of an activated carbon catalyst to prepare the target product 2,3,3,3-tetrafluoropropene. The process is complex in reaction steps and unfavorable to industrial production, and has the problems of low conversion rate, high reaction temperature and the like.

A United States patent US20090099396 discloses a method for preparing 2,3,3,3-tetrafluoropropene by reactions in two steps with 1,1,2,3-tetrachloropropene as a raw material. The method includes the following reaction steps: (1) subjecting 1,1,2,3-tetrachloropropene and HF to a liquid phase fluorination reaction with SbCl₅ as a catalyst to prepare 1,1,1,2,3-pentafluoropropane (HFC-245eb), wherein the conversion rate of TCP can reach 100%, but the selectivity of HFC-245eb is only 53-59%, and many by-products are produced; and (2) subjecting the HFC-245eb to a liquid phase dehydrofluorination reaction under the action of an alkali metal hydroxide to produce the target product 2,3,3,3-tetrafluoropropene. The process has the advantages of few reaction steps and low investment in equipment. However, the intermediate product HFC-245eb has low selectivity, and the by-products are difficult to separate.

### 3. Trifluoropropene route:

A patent CN101979364A discloses a method for preparing 2,3,3,3-tetrafluoropropene with 3,3,3-trifluoropropene as a raw material. The method includes the following four reaction steps: (1) subjecting 3,3,3-trifluoropropene and chlorine to an addition reaction under the action of photocatalysis to produce 1,2-dichloro-3,3,3-trifluoropropane, wherein the conversion rate of the raw material reaches 95%, and the selectivity reaches 90%; (2) subjecting the 1,2-dichloro-3,3,3-trifluoropropane to a liquid phase dehydrochlorination reaction under the action of an alkali metal hydroxide to produce 2-chloro-3,3,3-trifluoropropene (HCFO-1233xf), wherein the conversion rate and the selectivity both reach 90%; (3) subjecting the HCFO-1233xf and HF to an addition reaction to produce 2-chloro-1,1,1,2-tetrafluoropropane (HCFC-244bb), wherein a catalyst is SnCl₄, TiCl₄ or fluorosulfonic acid, the conversion rate of the raw material reaches 95%, and the selectivity is 90-96%; and (4) subjecting the HCFC-244bb to a liquid phase dehydrochlorination reaction under the action of an alkali metal catalyst to prepare a target product CF₃CF=CH₂, wherein the conversion rate of the raw material is 95%, and the selectivity is 90-95%. The process has a long synthetic route, a chlorination reaction in the first step has high requirements for equipment, more waste liquid is produced by dehalogenation reactions in two steps, and the reactions have a low total yield and a high synthetic cost.

### 4. Other routes:

Document WO2019/003896 A1 discloses the dehydrochlorination of 3-chloro-1,1,1,2-tetrafluoropropane to yield 2,3,3,3-tetrafluoropropene. The 3-chloro-1,1,1,2-tetrafluoropropane is produced via reaction of 1,1,1,2-tetrafluoropropane with chlorine. An Asahi patent WO2011162341A discloses a method for preparing 2,3,3,3-tetrafluoropropene by hydrogenation reduction with 1,1-dichloro-2,3,3,3-tetrafluoropropene (CFO-1214ya) as a raw material under the action of a palladium catalyst. However, by means of the method, the hydrogenation reduction reaction degree is difficult to control, and intermediates or over-reduction products such as 1-chloro-2,3,3,3-tetrafluoropropene (HCFO-1224yd), 1-chloro-2,3,3,3-tetrafluoropropane (HCFC-244eb) and 2,3,3,3-tetrafluoropropane (HFC-254eb) are easily produced. The method has low product selectivity and complex post-treatment operations. In addition, the by-product HFC-254eb is prone to a further dehydrofluorination reaction during alkali washing treatment to produce 3,3,3-trifluoropropene (HFO-1243zf) having a boiling point similar to that of HFO-1234yf, thereby further increasing the difficulty in separation of impurities. Although the above problems can be reduced by controlling the reaction temperature of a catalyst bed layer and the absorption temperature of alkali washing, the problems are not reduced obviously. Moreover, process conditions are difficult to control, and the method is not suitable for industrial amplification.

### SUMMARY

In order to solve the above technical problems, the present disclosure provides a two-step method for preparing 2,3,3,3-tetrafluoropropene, which has a simple process, mild reaction conditions and high product selectivity and is suitable for industrial production.

The object of the present disclosure is realized through the following technical schemes.

The present disclosure provides a two-step method for preparing 2,3,3,3-tetrafluoropropene. The method includes:
A1, a telomerization step: subjecting chlorofluoromethane and trifluoroethylene to a pressure telomerization reaction under the action of a telomerization catalyst to prepare 3-chloro-1,1,1,2-tetrafluoropropane, wherein the telomerization catalyst is a Lewis acid catalyst or a mixed catalyst of a Lewis acid catalyst and dichloromethane; and
A2, a dehydrochlorination step: subjecting the 3-chloro-1,1,1,2-tetrafluoropropane to dehydrochlorination under the catalytic action of activated carbon to obtain 2,3,3,3-tetrafluoropropene.

The two-step method for preparing 2,3,3,3-tetrafluoropropene in the present disclosure has a reaction equation as follows:

CH₂FCl+CF₂=CHF→CF₃CHFCH₂Cl→CF₃CF=CH₂.

The Lewis acid catalyst of the present disclosure is selected from at least one halide of Al, Sb, Ti, Zr and Hf. As a preference, the Lewis acid catalyst is selected from at least one of ZrCl₄, HfCl₄, TiCl₄, AlCl₃, AlF₃ and SbF₅. More preferably, the Lewis acid catalyst is ZrCl₄ or HfCl₄.

The raw materials, chlorofluoromethane and trifluoroethylene, of the present disclosure are subjected to the telomerization reaction under pressure conditions, and the raw material, chlorofluoromethane, is partially or completely converted into liquid under reaction conditions. In addition, the 3-chloro-1,1,1,2-tetrafluoropropane produced by the telomerization reaction is liquid. Therefore, a solvent-free reaction is preferably used in the step A1 of the present disclosure to reduce a separation step of an intermediate and/or a product.

The telomerization catalyst of the present disclosure may be a single Lewis acid catalyst or a mixed catalyst of a Lewis acid catalyst and dichloromethane. When a mixed catalyst is used, the Lewis acid catalyst dissociates and activates the chlorofluoromethane to form F⁻, CH₂Cl⁺, Cl⁻, CH₂F⁺ and other ions; and the dichloromethane inhibits the dissociated F⁻, CH₂Cl⁺, Cl⁻, CH₂F⁺ and other ions from rebinding, so as to ensure that the F⁻ and CH₂Cl⁺ ions undergo a directed telomerization reaction with the trifluoroethylene to obtain a telomerization product CF₃CHFCH₂Cl with high selectivity.

In a chemical reaction, the ratio of raw materials, the ratio of raw materials to a catalyst, the reaction temperature, the reaction time and the like will affect reaction results. In particular, combinations of multiple variables will have a great impact on reaction results.

In the telomerization step of the present disclosure, the molar ratio of the chlorofluoromethane to the trifluoroethylene is 1:0.1 to 1:10; and more preferably, the molar ratio of the chlorofluoromethane to the trifluoroethylene is 1:1 to 1:5. The amount of the Lewis acid catalyst is 0.01 to 50 wt% of the mass of the chlorofluoromethane; and more preferably, the amount of the Lewis acid catalyst is 0.1 to 10 wt% of the mass of the chlorofluoromethane. When a mixed catalyst of a Lewis acid catalyst and dichloromethane is used, the molar ratio of the dichloromethane to the chlorofluoromethane is 1:0.01 to 1:10; and more preferably, the molar ratio of the dichloromethane to the chlorofluoromethane is 1:0.1 to 1:5.

The telomerization step of the present disclosure is carried out under pressure conditions at a reaction temperature of -30°C to 100°C and a reaction pressure of 0.5 to 5.0 MPa for a reaction time of 1 to 50 h. More preferably, the reaction temperature is 0 to 50°C, the reaction pressure is 0.8 to 3.0 MPa, and the reaction time is 5 to 10 h.

The dehydrochlorination step of the present disclosure is carried out under the catalytic action of activated carbon, and the activated carbon is selected from fruit shell type activated carbon, coal type activated carbon or wood type activated carbon and is preferably fruit shell type activated carbon.

The dehydrochlorination step is carried out at a reaction temperature of 200 to 500°C, and preferably, the reaction temperature is 300-350°C.

In order to further improve the purity of a 2,3,3,3-tetrafluoropropene product and reduce post-treatment difficulty, the 3-chloro-1,1,1,2-tetrafluoropropane obtained in the telomerization step is used in the dehydrochlorination step after rectification and separation.

In a second aspect, the present disclosure provides a method for co-producing 2,3,3,3-tetrafluoropropene and 1-chloro-2,3,3,3-tetrafluoropropene. The method for co-producing 2,3,3,3-tetrafluoropropene and 1-chloro-2,3,3,3-tetrafluoropropene includes:
A1, a telomerization step: subjecting chlorofluoromethane and trifluoroethylene to a pressure telomerization reaction under the action of a telomerization catalyst to prepare 3-chloro-1,1,1,2-tetrafluoropropane, wherein the telomerization catalyst is a Lewis acid catalyst or a mixed catalyst of a Lewis acid catalyst and dichloromethane; and
A2, a removal step: subjecting the 3-chloro-1,1,1,2-tetrafluoropropane to a dehydrochlorination reaction and a dehydrogenation reaction simultaneously under the action of an activated carbon supported noble metal catalyst to obtain 2,3,3,3-tetrafluoropropene and 1-chloro-2,3,3,3-tetrafluoropropene, wherein the activated carbon supported noble metal catalyst is at least one of Pd/activated carbon (Pd/AC) and Pt/activated carbon (Pt/AC).

The method for co-producing 2,3,3,3-tetrafluoropropene and 1-chloro-2,3,3,3-tetrafluoropropene in the present disclosure has a reaction equation as follows:

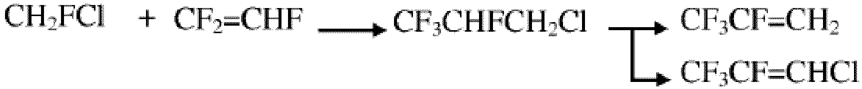

The Lewis acid catalyst of the present disclosure is selected from at least one halide of Al, Sb, Ti, Zr and Hf. As a preference, the Lewis acid catalyst is selected from at least one of ZrCl₄, HfCl₄, TiCl₄, AlF₃, AlCl₃ and SbF₅. More preferably, the Lewis acid catalyst is ZrCl₄ or HfCl₄.

The raw materials, chlorofluoromethane and trifluoroethylene, of the present disclosure are subjected to the telomerization reaction under pressure conditions, and the raw material, chlorofluoromethane, is partially or completely converted into liquid under reaction conditions. In addition, the 3-chloro-1,1,1,2-tetrafluoropropane produced by the telomerization reaction is liquid. Therefore, a solvent-free reaction is preferably used in the step A1 of the present disclosure to reduce a separation step of an intermediate and/or a product.

The telomerization catalyst of the present disclosure may be a single Lewis acid catalyst or a mixed catalyst of a Lewis acid catalyst and dichloromethane. When a mixed catalyst is used, the Lewis acid catalyst dissociates and activates the chlorofluoromethane to form F⁻, CH₂Cl⁺, Cl⁻, CH₂F⁺ and other ions; and the dichloromethane inhibits the dissociated F⁻, CH₂Cl⁺, Cl⁻, CH₂F⁺ and other ions from rebinding, so as to ensure that the F⁻ and CH₂Cl⁺ ions undergo a directed telomerization reaction with the trifluoroethylene to obtain a telomerization product CF₃CHFCH₂Cl with high selectivity.

In a chemical reaction, the ratio of raw materials, the ratio of raw materials to a catalyst, the reaction temperature, the reaction time and the like will affect reaction results. In particular, combinations of multiple variables will have a great impact on reaction results.

In the telomerization step of the present disclosure, the molar ratio of the chlorofluoromethane to the trifluoroethylene is 1:0.1 to 1:10; and more preferably, the molar ratio of the chlorofluoromethane to the trifluoroethylene is 1:1 to 1:5. The amount of the Lewis acid catalyst is 0.01 to 50 wt% of the mass of the chlorofluoromethane; and more preferably, the amount of the Lewis acid catalyst is 0.1 to 10 wt% of the mass of the chlorofluoromethane. When a mixed catalyst of a Lewis acid catalyst and dichloromethane is used, the molar ratio of the dichloromethane to the chlorofluoromethane is 1:0.01 to 1:10; and more preferably, the molar ratio of the dichloromethane to the chlorofluoromethane is 1:0.1 to 1:5.

The telomerization step of the present disclosure is carried out under pressure conditions at a reaction temperature of -30°C to 100°C and a reaction pressure of 0.5 to 5.0 MPa for a reaction time of 1 to 50 h. More preferably, the reaction temperature is 0 to 50°C, the reaction pressure is 0.8 to 3.0 MPa, and the reaction time is 5 to 10 h.

In the removal step of the present disclosure, under the action of the activated carbon supported noble metal catalyst, the raw material, 3-chloro-1,1,1,2-tetrafluoropropane, is subjected to the dehydrochlorination reaction when adsorbed to activated carbon and is subjected to the dehydrogenation reaction when adsorbed to a noble metal site on the activated carbon, so as to obtain the 2,3,3,3-tetrafluoropropene and the 1-chloro-2,3,3,3-tetrafluoropropene at the same time.

The activated carbon supported noble metal catalyst may be prepared by a conventional method in a case that the activated carbon supported noble metal catalyst of the present disclosure can be obtained. As a preference, the activated carbon supported noble metal catalyst of the present disclosure is prepared by an impregnation method. The impregnation method includes the following steps:
B1, pretreatment of a carrier: drying activated carbon at 90 to 120°C for 12 h or above;
B2, impregnation in a metal salt: impregnating the pretreated activated carbon in a soluble salt solution of Pd or Pt under vacuum or atmospheric pressure conditions;
B3, drying the impregnated activated carbon at a temperature of 90 to 120°C for 12 h or above; and
B4, reducing the dried activated carbon by a mixed gas of hydrogen and nitrogen to obtain the activated carbon supported noble metal catalyst, wherein the hydrogen has a volume ratio of 5 to 50% in the mixed gas of hydrogen and nitrogen, and the reducing is carried out at a temperature of 150 to 300°C.

In the activated carbon supported noble metal catalyst, the Pd or the Pt has a supporting capacity of 0.1 to 5.0 wt%, preferably 0.5-1.5 wt%.

A gas-solid reaction is carried out in the removal step of the present disclosure, the 3-chloro-1,1,1,2-tetrafluoropropane is vaporized and then loaded onto a catalyst bed layer by nitrogen to carry out a removal reaction, the removal reaction has a material volume space velocity of 50 to 300 h⁻¹, and the volume ratio of N₂ to the 3-chloro-1,1,1,2-tetrafluoropropane is (0.5-3.0):1, preferably (1.5-2.0):1.

The removal step of the present disclosure is carried out at a reaction temperature of 300 to 600°C, and preferably, the reaction temperature is 400-450°C.

Distribution of products obtained in the removal step A2 may be adjusted in a certain range by adjusting the preparation process of the activated carbon supported noble metal catalyst, the supporting capacity of a noble metal in the catalyst and reaction conditions. Generally, 30-90% of the 2,3,3,3-tetrafluoropropene and 10 to 50% of the 1-chloro-2,3,3,3-tetrafluoropropene are obtained in the removal step A2; and preferably, products obtained in the removal step include 50-60% of the 2,3,3,3-tetrafluoropropene, 30 to 50% of the 1-chloro-2,3,3,3-tetrafluoropropene and remaining by-products, such as 1-chloro-3,3,3-trifluoropropene.

In order to further reduce post-treatment difficulty, the 3-chloro-1,1,1,2-tetrafluoropropane obtained in the telomerization step is used in the removal step after rectification and separation.

In a third aspect, the present disclosure further provides a method for co-producing 2,3,3,3-tetrafluoropropene and 1-chloro-3,3,3-trifluoropropene. The method for co-producing 2,3,3,3-tetrafluoropropene and 1-chloro-3,3,3-trifluoropropene includes:
A1, a telomerization step: subjecting chlorofluoromethane and trifluoroethylene to a pressure telomerization reaction under the action of a telomerization catalyst to prepare 3-chloro-1,1,1,2-tetrafluoropropane, wherein the telomerization catalyst is a Lewis acid catalyst or a mixed catalyst of a Lewis acid catalyst and dichloromethane; and
A2, a dehydrohalogenation step: subjecting the 3-chloro-1,1,1,2-tetrafluoropropane to a dehydrochlorination reaction and a dehydrofluorination reaction simultaneously under the action of a composite dehalogenation catalyst to obtain 2,3,3,3-tetrafluoropropene and 1-chloro-3,3,3-trifluoropropene, wherein the composite dehalogenation catalyst is prepared from at least one oxide or fluoride of Al, Mg or Cr and activated carbon powder.

The at least one oxide or fluoride of Al, Mg or Cr is selected from at least one of Al₂O₃, AlF₃, MgF₂ and Cr₂O₃; and the activated carbon powder is selected from fruit shell type activated carbon, coal type activated carbon or wood type activated carbon.

The method for co-producing 2,3,3,3-tetrafluoropropene and 1-chloro-3,3,3-trifluoropropene in the present disclosure has a reaction equation as follows:

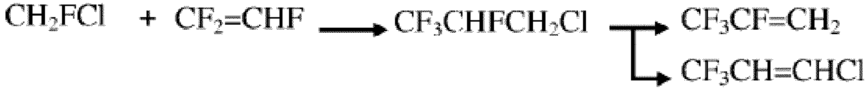

The Lewis acid catalyst of the present disclosure is selected from at least one halide of Al, Sb, Ti, Zr and Hf. As a preference, the Lewis acid catalyst is selected from at least one of ZrCl₄, HfCl₄, TiCl₄, AlF₃, AlCl₃ and SbF₅. More preferably, the Lewis acid catalyst is ZrCl₄ or HfCl₄.

The raw materials, chlorofluoromethane and trifluoroethylene, of the present disclosure are subjected to the telomerization reaction under pressure conditions, and the raw material, chlorofluoromethane, is partially or completely converted into liquid under reaction conditions. In addition, the 3-chloro-1,1,1,2-tetrafluoropropane produced by the telomerization reaction is liquid. Therefore, a solvent-free reaction is preferably used in the step A1 of the present disclosure to reduce a separation step of an intermediate and/or a product.

The telomerization catalyst of the present disclosure may be a single Lewis acid catalyst or a mixed catalyst of a Lewis acid catalyst and dichloromethane. When a mixed catalyst is used, the Lewis acid catalyst dissociates and activates the chlorofluoromethane to form F⁻, CH₂Cl⁺, Cl⁻, CH₂F⁺ and other ions; and the dichloromethane inhibits the dissociated F⁻, CH₂Cl⁺, Cl⁻, CH₂F⁺ and other ions from rebinding, so as to ensure that the F⁻ and CH₂Cl⁺ ions undergo a directed telomerization reaction with the trifluoroethylene to obtain a telomerization product CF₃CHFCH₂Cl with high selectivity.

In a chemical reaction, the ratio of raw materials, the ratio of raw materials to a catalyst, the reaction temperature, the reaction time and the like will affect reaction results. In particular, combinations of multiple variables will have a great impact on reaction results.

In the telomerization step of the present disclosure, the molar ratio of the chlorofluoromethane to the trifluoroethylene is 1:0.1 to 1:10; and more preferably, the molar ratio of the chlorofluoromethane to the trifluoroethylene is 1:1 to 1:5. The amount of the Lewis acid catalyst is 0.01 to 50 wt% of the mass of the chlorofluoromethane; and more preferably, the amount of the Lewis acid catalyst is 0.1 to 10 wt% of the mass of the chlorofluoromethane. When a mixed catalyst of a Lewis acid catalyst and dichloromethane is used, the molar ratio of the dichloromethane to the chlorofluoromethane is 1:0.01 to 1:10; and more preferably, the molar ratio of the dichloromethane to the chlorofluoromethane is 1:0.1 to 1:5.

The telomerization step of the present disclosure is carried out under pressure conditions at a reaction temperature of -30°C to 100°C and a reaction pressure of 0.5 to 5.0 MPa for a reaction time of 1 to 50 h. More preferably, the reaction temperature is 0 to 50°C, the reaction pressure is 0.8 to 3.0 MPa, and the reaction time is 5 to 10 h.

In the dehydrohalogenation step of the present disclosure, under the action of the composite dehalogenation catalyst, the 3-chloro-1,1,1,2-tetrafluoropropane is subjected to the dehydrochlorination reaction when adsorbed to activated carbon and is subjected to the dehydrofluorination reaction when adsorbed to Al₂O₃ and/or AlF₃ and/or MgF₂ and/or Cr₂O₃, so as to obtain the 2,3,3,3-tetrafluoropropene and the 1-chloro-3,3,3-trifluoropropene at the same time.

The composite dehalogenation catalyst of the present disclosure may be prepared by a conventional method in a case that the composite dehalogenation catalyst of the present disclosure can be obtained. As a preference, the composite dehalogenation catalyst is prepared by a co-blending method. The co-blending method includes the following steps:
B1, mixing: blending Al₂O₃ and/or AlF₃ and/or MgF₂ and/or Cr₂O₃ with activated carbon powder at a mass ratio of (0.01-0.25):1 and performing thorough mixing by a mechanical stirring mode or a ball milling mode;
B2, sifting: sifting the mixed material to remove an unevenly mixed part;
B3, molding: transferring the sifted material to a tablet press for compression molding; and
B4, drying a molded catalyst to prepare the composite dehalogenation catalyst, such as Al₂O₃₋AC, AlF₃-AC, MgF₂-AC, Cr₂O₃-AC and other catalysts.

In the molding step B3, the catalyst may be prepared in a columnar shape, a sheet shape and other shapes, and the specific shape is not limited.

In the step B4, the drying is usually carried out at 90 to 120°C for 12h or above.

As for the composite dehalogenation catalyst of the present disclosure, when the Al₂O₃ is co-blended with the activated carbon powder, the content of the Al₂O₃ is 1.0 to 20 wt% of the total amount of the catalyst; when the AlF₃ is co-blended with the activated carbon powder, the content of the AlF₃ is 1.0 to 20 wt% of the total amount of the catalyst; when the MgF₂ is co-blended with the activated carbon powder, the content of the MgF₂ is 1.0 to 20 wt% of the total amount of the catalyst; and when the Cr₂O₃ is co-blended with the activated carbon powder, the content of the Cr₂O₃ is 1.0 to 20 wt% of the total amount of the catalyst.

A gas-solid reaction is carried out in the dehydrohalogenation step of the present disclosure, the 3-chloro-1,1,1,2-tetrafluoropropane is vaporized and then loaded onto a catalyst bed layer by nitrogen to carry out a dehydrohalogenation reaction, the dehydrohalogenation reaction has a material volume space velocity of 50 to 300 h⁻¹, and the volume ratio of N₂ to the 3-chloro-1,1,1,2-tetrafluoropropane is (0.5-3.0):1, preferably (1.5-2.0):1.

The dehydrohalogenation step of the present disclosure is carried out at a reaction temperature of 300 to 500°C, and preferably, the reaction temperature is 350-450°C.

Distribution of products obtained in the dehydrohalogenation step may be adjusted in a certain range by adjusting the preparation process of the composite dehalogenation catalyst, contents of active components in the catalyst and reaction conditions. Generally, 10 to 50% of the 2,3,3,3-tetrafluoropropene and 10-70% of the 1-chloro-3,3,3-trifluoropropene are obtained in the dehydrohalogenation step; and preferably, products obtained in the dehydrohalogenation step include 20-40% of the 2,3,3,3-tetrafluoropropene, 30-60% of the 1-chloro-3,3,3-trifluoropropene and remaining unknown by-products.

In order to further reduce post-treatment difficulty, the 3-chloro-1,1,1,2-tetrafluoropropane obtained in the telomerization step is used in the dehydrohalogenation step after rectification and separation.

Compared with the prior art, the present disclosure has the following beneficial effects.

According to the present disclosure, chlorofluoromethane and trifluoroethylene are used as raw materials and subjected to pressure telomerization under the action of a Lewis acid catalyst or a mixed catalyst of a Lewis acid catalyst and dichloromethane to obtain 3-chloro-1,1,1,2-tetrafluoropropane. The 3-chloro-1,1,1,2-tetrafluoropropane is prepared into 2,3,3,3-tetrafluoropropene under the catalytic action of activated carbon; or, the 3-chloro-1,1,1,2-tetrafluoropropane is subjected to a dehydrochlorination reaction and a dehydrogenation reaction simultaneously under the catalytic action of an activated carbon supported noble metal catalyst to co-produce 2,3,3,3-tetrafluoropropene and 1-chloro-2,3,3,3-tetrafluoropropene; or, the 3-chloro-1,1,1,2-tetrafluoropropane is subjected to a dehydrochlorination reaction and a dehydrofluorination reaction simultaneously under the catalytic action of a composite dehalogenation catalyst to co-produce 2,3,3,3-tetrafluoropropene and 1-chloro-3,3,3-trifluoropropene. The three methods for preparing 2,3,3,3-tetrafluoropropene provided by the present disclosure have the advantages of a simple process, mild reaction conditions, high selectivity of a telomerization product and a target product and the like, and are suitable for industrial amplification.

### Detailed Description of the Embodiments

The present disclosure is further described below in combination with specific embodiments, but the scope of the claims is not limited to these specific embodiments.

A first aspect of the embodiments of the present disclosure is to provide a two-step method for preparing 2,3,3,3-tetrafluoropropene.

### Example 1.1

The present example provides a two-step method for preparing 2,3,3,3-tetrafluoropropene. The method includes a telomerization step and a dehydrochlorination step and is specifically as follows.

### 1. Telomerization step

A1. An autoclave made of an Inconel alloy with a volume of 250 mL was used as a reactor. 3.0 g of HfCl₄ and 20.0 g of dichloromethane were separately added into the reactor, then the reactor was sealed, and 1.0 MPa of nitrogen was repeatedly introduced to replace air in the reactor for three times.

A2. After the air in the reactor was completely replaced, 19.9 g (0.29 mol) of chlorofluoromethane and 24.6 g (0.30 mol) of trifluoroethylene were sequentially introduced.

A3. The reaction temperature was set at 10°C, the stirring rate was set at 300 rpm, and the initial reaction pressure was set at 0.9 MPa. With progressing of a reaction, the pressure was gradually decreased, and the reaction time was 10 h.

A4. After the reaction was completed, unreacted gas phase raw materials including the trifluoroethylene and/or the chlorofluoromethane and small amounts of a telomerization product and the dichloromethane were collected. The materials in the reactor were subjected to solid-liquid separation treatment such as filtration or distillation, wherein a Lewis acid catalyst (HfCl₄) was used as a solid part, and the dichloromethane and the telomerization product were used as liquid phase materials. Then, rectification and separation were carried out to obtain 3-chloro-1,1,1,2-tetrafluoropropane with a purity of 99.9% for use in a dehydrochlorination reaction.

The unreacted gas phase raw materials and the separated Lewis acid catalyst were transferred back to the telomerization step for reuse.

According to analysis of the gas phase and liquid phase materials by gas chromatography, the conversion rate of chlorofluoromethane was 76.5%, the selectivity of 3-chloro-1,1,1,2-tetrafluoropropane was 81.2%, 1-chloro-1,1,2,3-tetrafluoropropane was a main by-product with a selectivity of 15.3%, and few other by-products were produced.

### 2. Dehydrochlorination step

B1. A reaction tube made of an Inconel alloy with an inner diameter of 19 mm and a length of 800 mm was used as a fixed bed reactor. Coconut shell type activated carbon with a volume of 20 mL and a particle size of 10-20 mesh was filled to the middle of the fixed bed reactor, a reaction pipeline was connected, and nitrogen was introduced for purging at a flow rate of 100 mL/min.

B2. The reaction temperature was set at 350°C, and the reactor was heated at a heating rate of 5°C/min.

B3. After a catalyst bed layer reached the reaction temperature, the nitrogen flow rate was adjusted to 20 mL/min. Meanwhile, the 3-chloro-1,1,1,2-tetrafluoropropane with a purity of 99.9% was continuously introduced into the fixed bed reactor at a rate of 5.0 g/h to carry out a reaction.

B4. According to analysis of a gas mixture flowing out of the reactor by on-line gas chromatography (GC) and gas chromatography-mass spectrometry (GC/MS), the conversion rate of 3-chloro-1,1,1,2-tetrafluoropropane was 99.6%, and the selectivity of a 2,3,3,3-tetrafluoropropene product was 99.3%.

### Example 1.2

The present example provides a method for preparing 2,3,3,3-tetrafluoropropene. The method has the same operations as that in Example 1.1, and only has the differences that in the telomerization step, 4.0 g of ZrCl₄ was used to replace the HfCl₄, the amount of chlorofluoromethane was increased to 39.7 g (0.58 mol) and the amount of trifluoroethylene was increased to 71.3 g (0.87 mol) while other conditions were remained unchanged.

According to analysis of gas phase and liquid phase materials in the telomerization step by gas chromatography, the conversion rate of chlorofluoromethane was 99.0%, the selectivity of 3-chloro-1,1,1,2-tetrafluoropropane was 89.9%, 1-chloro-1,1,2,3-tetrafluoropropane was a main by-product with a selectivity of 5.3%, and few other by-products were produced.

### Example 1.3

The present example provides a method for preparing 2,3,3,3-tetrafluoropropene. The method has the same operations as that in Example 1.2, and only has the differences that in the telomerization step, the dichloromethane was not used, the amount of trifluoroethylene was increased to 95.1 g (1.16 mol), meanwhile, the reaction temperature was increased to 30°C and the initial reaction pressure was increased to 1.5 MPa while other conditions were remained unchanged.

According to analysis of gas phase and liquid phase materials in the telomerization step by gas chromatography, the conversion rate of chlorofluoromethane was 99.5%, the selectivity of 3-chloro-1,1,1,2-tetrafluoropropane was 88.1%, 1-chloro-1,1,2,3-tetrafluoropropane was a main by-product with a selectivity of 4.1%, and few other by-products were produced.

### Example 1.4

The present example provides a method for preparing 2,3,3,3-tetrafluoropropene. The method has the same operations as that in Example 1.2, and only has the differences that in the telomerization step, 4.0 g, the same use amount, of AlCl₃ was used to replace the ZrCl₄, the dichloromethane was not used and the amount of trifluoroethylene was decreased to 52.5 g (0.64 mol) while other conditions were remained unchanged.

According to analysis of gas phase and liquid phase materials in the telomerization step by gas chromatography, the conversion rate of chlorofluoromethane was 99.6%, the selectivity of 3-chloro-1,1,1,2-tetrafluoropropane was 75.5%, 1-chloro-1,1,2,3-tetrafluoropropane was a main by-product with a selectivity of 15.9%, and few other by-products were produced.

### Example 1.5

The present example provides a method for preparing 2,3,3,3-tetrafluoropropene. The method has the same operations as that in Example 1.1, and only has the differences that in the step A2 of the telomerization step, after the chlorofluoromethane and the trifluoroethylene were sequentially introduced into the autoclave, high-purity and high-pressure nitrogen was used to perform pressure treatment on the autoclave so as to increase the pressure in the autoclave from 0.9 MPa to 3.0 MPa while other conditions were remained unchanged.

According to analysis of gas phase and liquid phase materials in the telomerization step by gas chromatography, the conversion rate of chlorofluoromethane was 99.8%, the selectivity of 3-chloro-1,1,1,2-tetrafluoropropane was 88.6%, 1-chloro-1,1,2,3-tetrafluoropropane was a main by-product with a selectivity of 7.6%, and few other by-products were produced.

### Example 1.6

The present example provides a method for preparing 2,3,3,3-tetrafluoropropene. The method has the same operations as that in Example 1.1, and only has the difference that in the dehydrochlorination step, 10- to 20-mesh coal type activated carbon was used to replace the coconut shell type activated carbon.

According to analysis of a dehydrochlorination product by chromatography, the conversion rate of 3-chloro-1,1,1,2-tetrafluoropropane was 99.2%, and the selectivity of a 2,3,3,3-tetrafluoropropene product reached 95.1%.

### Example 1.7

The present example provides a method for preparing 2,3,3,3-tetrafluoropropene. The method has the same operations as that in Example 1.1, and only has the difference that in the dehydrochlorination step, the reaction temperature was lowered to 300°C.

According to analysis of a dehydrochlorination product by chromatography, the conversion rate of 3-chloro-1,1,1,2-tetrafluoropropane was 75.8%, and the selectivity of a 2,3,3,3-tetrafluoropropene product was 99.2%.

### Example 1.8

The present example provides a method for preparing 2,3,3,3-tetrafluoropropene. The method has the same operations as that in Example 1.1, and only has the difference that in the dehydrochlorination step, the reaction temperature was lowered to 320°C.

According to analysis of a dehydrochlorination product by chromatography, the conversion rate of 3-chloro-1,1,1,2-tetrafluoropropane was 86.9%, and the selectivity of a 2,3,3,3-tetrafluoropropene product was 99.1%.

### Comparative Example 1.1

The present comparative example provides a method for preparing 2,3,3,3-tetrafluoropropene. The method has the same operations as that in Example 1.1, and only has the difference that 20.0 g of trichloromethane was used to replace the dichloromethane while other conditions were remained unchanged.

According to analysis of materials obtained by a reaction in the telomerization step by chromatography, the conversion rate of chlorofluoromethane was 86.9%, the selectivity of 3-chloro-1,1,1,2-tetrafluoropropane was 46.2%, a large amount of dichloromethane, as a disproportionation product of the chlorofluoromethane, was produced with a selectivity of 40.3%, and few other telomerization by-products were produced.

### Comparative Example 1.2

The present comparative example provides a method for preparing 2,3,3,3-tetrafluoropropene. The method has the same operations as that in Example 1.1, and only has the difference that 3.0 g of ZnCl₂ was used to replace the HfCl₄ while other conditions were remained unchanged.

According to analysis of materials obtained by a reaction in the telomerization step by chromatography, the conversion rate of chlorofluoromethane was 20.8%, and a target product, 3-chloro-1,1,1,2-tetrafluoropropane, was not produced.

### Comparative Example 1.3

The present comparative example provides a method for preparing 2,3,3,3-tetrafluoropropene. The method has the same operations as that in Example 1.1, and only has the difference that the HfCl₄ and the dichloromethane were not added while other conditions were remained unchanged.

According to analysis of materials obtained by a reaction in the telomerization step by chromatography, the conversion rate of chlorofluoromethane was 7.7%, a target product, 3-chloro-1,1,1,2-tetrafluoropropane, was not produced, and only a small amount of dichloromethane, as a disproportionation product of the chlorofluoromethane, was produced.

A second aspect of the embodiments of the present disclosure is to provide a method for co-producing 2,3,3,3-tetrafluoropropene and 1-chloro-2,3,3,3-tetrafluoropropene.

### Preparative Example 2.1

6.0 mL of a chloropalladic acid solution (with a concentration of 0.033 g Pd/mL) was taken and added into 80.0 mL of distilled water for uniform dilution to obtain an impregnation solution. 20.0 g of activated carbon pretreated by drying at 120°C for 12 h was taken and added into the impregnation solution for impregnation for 12 h or above, followed by drying at 120°C for 12 h to obtain a 1 wt% Pd/AC catalyst, recorded as cat 2.1.

### Preparative Example 2.2

9.2 mL of a chloropalladic acid solution (with a concentration of 0.033 g Pd/mL) was taken and added into 80.0 mL of distilled water for uniform dilution to obtain an impregnation solution. 20.0 g of activated carbon pretreated by drying at 120°C for 12 h was taken and added into the impregnation solution for impregnation for 12 h or above, followed by drying at 120°C for 12 h to obtain a 1.5 wt% Pd/AC catalyst, recorded as cat 2.2.

### Preparative Example 2.3

0.35 g of PtCl₄ was taken and dissolved in 80.0 mL of distilled water to obtain an impregnation solution. 20.0 g of activated carbon pretreated by drying at 120°C for 12 h was taken and added into the impregnation solution for impregnation for 12 h or above, followed by drying at 120°C for 12 h to obtain a 1 wt% Pt/AC catalyst, recorded as cat 2.3.

### Preparative Example 2.4

0.52 g of PtCl₄ was taken and dissolved in 80.0 mL of distilled water to obtain an impregnation solution. 20.0 g of activated carbon pretreated by drying at 120°C for 12 h was taken and added into the impregnation solution for impregnation for 12 h or above, followed by drying at 120°C for 12 h to obtain a 1.5 wt% Pt/AC catalyst, recorded as cat 2.4.

### Example 2.1

The present example provides a method for co-producing 2,3,3,3-tetrafluoropropene and 1-chloro-2,3,3,3-tetrafluoropropene. The method includes a telomerization step and a removal step and is specifically as follows.

### 1. Telomerization step

A1. An autoclave made of an Inconel alloy with a volume of 250 mL was used as a reactor. 3.0 g of HfCl₄ and 20.0 g of dichloromethane were separately added into the reactor, then the reactor was sealed, and 1.0 MPa of nitrogen was repeatedly introduced to replace air in the reactor for three times.

A2. After the air in the reactor was completely replaced, 19.9 g (0.29 mol) of chlorofluoromethane and 24.6 g (0.30 mol) of trifluoroethylene were sequentially introduced.

A3. The reaction temperature was set at 10°C, the stirring rate was set at 300 rpm, and the initial reaction pressure was set at 0.9 MPa. With progressing of a reaction, the pressure was gradually decreased, and the reaction time was 10 h.

A4. After the reaction was completed, unreacted gas phase raw materials including the trifluoroethylene and/or the chlorofluoromethane and small amounts of a telomerization product and the dichloromethane were collected. The materials in the reactor were subjected to solid-liquid separation treatment such as filtration or distillation, wherein a Lewis acid catalyst (HfCl₄) was used as a solid part, and the dichloromethane and the telomerization product were used as liquid phase materials. Then, rectification and separation were carried out to obtain 3-chloro-1,1,1,2-tetrafluoropropane with a purity of 99.9% for use in the removal step.

The unreacted gas phase raw materials and the separated Lewis acid catalyst were transferred back to the telomerization step for reuse.

According to analysis of the gas phase and liquid phase materials by gas chromatography, the conversion rate of chlorofluoromethane was 76.5%, the selectivity of 3-chloro-1,1,1,2-tetrafluoropropane was 81.2%, 1-chloro-1,1,2,3-tetrafluoropropane was a main by-product with a selectivity of 15.3%, and few other by-products were produced.

### 2. Removal step

B1. A reaction tube made of an Inconel alloy with an inner diameter of 19 mm and a length of 800 mm was used as a fixed bed reactor. Cat 2.1 with a volume of 20 mL was filled to the middle of the fixed bed reactor, a reaction pipeline was connected, and nitrogen was introduced for purging at a flow rate of 100 mL/min.

B2. The reaction temperature was set at 450°C, and the reactor was heated at a heating rate of 5°C/min.

B3. After a catalyst bed layer reached the reaction temperature, the nitrogen flow rate was adjusted to 20 mL/min. Meanwhile, the 3-chloro-1,1,1,2-tetrafluoropropane with a purity of 99.9% was continuously introduced into the fixed bed reactor at a rate of 5.0 g/h by a peristaltic pump to carry out a reaction.

B4. A gas mixture flowing out of the reactor was subjected to heat preservation treatment, followed by analysis by on-line GC and GC/MS. The conversion rate of 3-chloro-1,1,1,2-tetrafluoropropane was 96.8%, the content of 2,3,3,3-tetrafluoropropene in the product was 56.3%, and the content of 1-chloro-2,3,3,3-tetrafluoropropene was 31.4%.

### Example 2.2

The present example provides a method for co-producing 2,3,3,3-tetrafluoropropene and 1-chloro-2,3,3,3-tetrafluoropropene. The method has the same operations as that in Example 2.1, and only has the differences that in the telomerization step, 4.0 g of ZrCl₄ was used to replace the HfCl₄, the amount of chlorofluoromethane was increased to 39.7 g (0.58 mol) and the amount of trifluoroethylene was increased to 71.3 g (0.87 mol) while other conditions were remained unchanged.

According to analysis of gas phase and liquid phase materials in the telomerization step by gas chromatography, the conversion rate of chlorofluoromethane was 99.0%, the selectivity of 3-chloro-1,1,1,2-tetrafluoropropane was 89.9%, 1-chloro-1,1,2,3-tetrafluoropropane was a main by-product with a selectivity of 5.3%, and few other by-products were produced.

### Example 2.3

The present example provides a method for co-producing 2,3,3,3-tetrafluoropropene and 1-chloro-2,3,3,3-tetrafluoropropene. The method has the same operations as that in Example 2.2, and only has the differences that in the telomerization step, the dichloromethane was not used, the amount of trifluoroethylene was increased to 95.1 g (1.16 mol), meanwhile, the reaction temperature was increased to 30°C and the initial reaction pressure was increased to 1.5 MPa while other conditions were remained unchanged.

According to analysis of gas phase and liquid phase materials in the telomerization step by gas chromatography, the conversion rate of chlorofluoromethane was 99.5%, the selectivity of 3-chloro-1,1,1,2-tetrafluoropropane was 88.1%, 1-chloro-1,1,2,3-tetrafluoropropane was a main by-product with a selectivity of 4.1%, and few other by-products were produced.

### Example 2.4

The present example provides a method for co-producing 2,3,3,3-tetrafluoropropene and 1-chloro-2,3,3,3-tetrafluoropropene. The method has the same operations as that in Example 2.2, and only has the differences that in the telomerization step, 4 g, the same use amount, of AlCl₃ was used to replace the ZrCl₄, the dichloromethane was not used and the amount of trifluoroethylene was decreased to 52.5 g (0.64 mol) while other conditions were remained unchanged.

According to analysis of gas phase and liquid phase materials in the telomerization step by gas chromatography, the conversion rate of chlorofluoromethane was 99.6%, the selectivity of 3-chloro-1,1,1,2-tetrafluoropropane was 75.5%, 1-chloro-1,1,2,3-tetrafluoropropane was a main by-product with a selectivity of 15.9%, and few other by-products were produced.

### Example 2.5

The present example provides a method for co-producing 2,3,3,3-tetrafluoropropene and 1-chloro-2,3,3,3-tetrafluoropropene. The method has the same operations as that in Example 2.1, and only has the differences that in the step A2 of the telomerization step, after the chlorofluoromethane and the trifluoroethylene were introduced into the autoclave in advance, high-purity and high-pressure nitrogen was used to perform pressure treatment on the autoclave so as to increase the pressure in the autoclave from 0.9 MPa to 3.0 MPa while other conditions were remained unchanged.

According to analysis of gas phase and liquid phase materials in the telomerization step by gas chromatography, the conversion rate of chlorofluoromethane was 99.8%, the selectivity of 3-chloro-1,1,1,2-tetrafluoropropane was 88.6%, 1-chloro-1,1,2,3-tetrafluoropropane was a main by-product with a selectivity of 7.6%, and few other by-products were produced.

### Example 2.6

The present example provides a method for co-producing 2,3,3,3-tetrafluoropropene and 1-chloro-2,3,3,3-tetrafluoropropene. The method has the same operations as that in Example 2.1, and only has the difference that in the removal step, cat 2.3 was used to replace the cat 2.1.

According to analysis of a removal reaction product by chromatography, the conversion rate of 3-chloro-1,1,1,2-tetrafluoropropane was 89.3%, the content of 2,3,3,3-tetrafluoropropene in the product was 90.3%, and the content of 1-chloro-2,3,3,3-tetrafluoropropene was 7.7%.

### Example 2.7

The present example provides a method for co-producing 2,3,3,3-tetrafluoropropene and 1-chloro-2,3,3,3-tetrafluoropropene. The method has the same operations as that in Example 2.1, and only has the difference that in the removal step, the amount of the cat 2.1 was increased to 40 mL.

According to analysis of a removal reaction product by chromatography, the conversion rate of 3-chloro-1,1,1,2-tetrafluoropropane was 95.8%, the content of 2,3,3,3-tetrafluoropropene in the product was 65.7%, and the content of 1-chloro-2,3,3,3-tetrafluoropropene was 26.8%.

### Example 2.8

The present example provides a method for co-producing 2,3,3,3-tetrafluoropropene and 1-chloro-2,3,3,3-tetrafluoropropene. The method has the same operations as that in Example 2.1, and only has the difference that in the removal step, cat 2.2 was used to replace the cat 2.1.

According to analysis of a removal reaction product by chromatography, the conversion rate of 3-chloro-1,1,1,2-tetrafluoropropane was 70.1%, the content of 2,3,3,3-tetrafluoropropene in the product was 39.2%, and the content of 1-chloro-2,3,3,3-tetrafluoropropene was 28.5%.

### Example 2.9

The present example provides a method for co-producing 2,3,3,3-tetrafluoropropene and 1-chloro-2,3,3,3-tetrafluoropropene. The method has the same operations as that in Example 2.1, and only has the difference that in the removal step, the reaction temperature was 400°C.

According to analysis of a removal reaction product by chromatography, the conversion rate of 3-chloro-1,1,1,2-tetrafluoropropane was 96.7%, the content of 2,3,3,3-tetrafluoropropene in the product was 85.2%, and the content of 1-chloro-2,3,3,3-tetrafluoropropene was 10.3%.

### Comparative Example 2.1

The present comparative example provides a method for co-producing 2,3,3,3-tetrafluoropropene and 1-chloro-2,3,3,3-tetrafluoropropene. The method has the same operations as that in Example 2.1, and only has the difference that 20.0 g of trichloromethane was used to replace the dichloromethane while other conditions were remained unchanged.

According to analysis of materials obtained by a reaction in the telomerization step by chromatography, the conversion rate of chlorofluoromethane was 86.9%, the selectivity of 3-chloro-1,1,1,2-tetrafluoropropane was 46.2%, a large amount of dichloromethane, as a disproportionation product of the chlorofluoromethane, was produced with a selectivity of 40.3%, and few other telomerization by-products were produced.

### Comparative Example 2.2

The present comparative example provides a method for co-producing 2,3,3,3-tetrafluoropropene and 1-chloro-2,3,3,3-tetrafluoropropene. The method has the same operations as that in Example 2.1, and only has the difference that 3.0 g of ZnCl₂ was used to replace the HfCl₄ while other conditions were remained unchanged.

According to analysis of materials obtained by a reaction in the telomerization step by chromatography, the conversion rate of chlorofluoromethane was 20.8%, and a target product, 3-chloro-1,1,1,2-tetrafluoropropane, was not produced.

### Comparative Example 2.3

The present comparative example provides a method for co-producing 2,3,3,3-tetrafluoropropene and 1-chloro-2,3,3,3-tetrafluoropropene. The method has the same operations as that in Example 2.1, and only has the difference that the HfCl₄ and the dichloromethane were not added while other conditions were remained unchanged.

According to analysis of materials obtained by a reaction in the telomerization step by chromatography, the conversion rate of chlorofluoromethane was 7.7%, a target product, 3-chloro-1,1,1,2-tetrafluoropropane, was not produced, and only a small amount of dichloromethane, as a disproportionation product of the chlorofluoromethane, was produced.

### Comparative Example 2.4

The present comparative example provides a method for co-producing 2,3,3,3-tetrafluoropropene and 1-chloro-2,3,3,3-tetrafluoropropene. The method has the same operations as that in Example 2.1, and only has the difference that in the removal step, activated carbon pretreated by drying at 120°C for 12 h was used to replace the cat 2.1 while other conditions were remained unchanged.

According to analysis of a removal reaction product by chromatography, the conversion rate of 3-chloro-1,1,1,2-tetrafluoropropane was 99.7%, the content of 2,3,3,3-tetrafluoropropene in the product was 99.0%, and 1-chloro-2,3,3,3-tetrafluoropropene was not produced.

### Comparative Example 2.5

The present comparative example provides a method for co-producing 2,3,3,3-tetrafluoropropene and 1-chloro-2,3,3,3-tetrafluoropropene. The method has the same operations as that in Example 2.1, and only has the difference that Al₂O₃ was used to replace the cat 2.1 while other conditions were remained unchanged.

According to analysis of a removal reaction product by chromatography, the conversion rate of 3-chloro-1,1,1,2-tetrafluoropropane was 50.1%, the content of 2,3,3,3-tetrafluoropropene in the product was 3.1%, the content of 1-chloro-3,3,3-trifluoropropene was 62.2%, and 1-chloro-2,3,3,3-tetrafluoropropene was not produced.

A third aspect of the embodiments of the present disclosure is to provide a method for co-producing 2,3,3,3-tetrafluoropropene and 1-chloro-3,3,3-trifluoropropene.

### Preparative Example 3.1

The present preparative example provides preparation of a Cr₂O₃-AC catalyst by co-blending Cr₂O₃ with activated carbon powder. The preparation includes the following steps:
S1, blending Cr₂O₃ and coconut shell type activated carbon powder at a mass ratio of 1/9, and placing the blended material into a ball mill for ball milling and mixing so as to achieve even dispersion of various components;
S2, sifting the mixed material to remove an unevenly mixed part;
S3, transferring the sifted material to a tablet press for compression molding to obtain a columnar catalyst; and
S4, drying the molded catalyst at 120°C for 12 h to prepare a Cr₂O₃-AC catalyst, recorded as cat 3.1.

### Preparative Example 3.2

The present preparative example has the same operations as that in Preparative Example 3.1, and only has the differences that AlF₃ was used to replace the Cr₂O₃, and an AlF₃-AC catalyst was prepared, which was recorded as cat 3.2.

### Preparative Example 3.3

The present preparative example has the same operations as that in Preparative Example 3.1, and only has the differences that the mass ratio of the Cr₂O₃ to the activated carbon was changed into 1/4, and a Cr₂O₃-AC catalyst was prepared, which was recorded as cat 3.3.

### Example 3.1

The present example provides a method for co-producing 2,3,3,3-tetrafluoropropene and 1-chloro-3,3,3-trifluoropropene. The method includes a telomerization step and a dehydrohalogenation step and is specifically as follows.

### 1. Telomerization step

A1. An autoclave made of an Inconel alloy with a volume of 250 mL was used as a reactor, 3.0 g of HfCl₄ and 20.0 g of dichloromethane were separately added into the reactor, then the reactor was sealed, and 1.0 MPa of nitrogen was repeatedly introduced to replace air in the reactor for three times.

A2. After the air in the reactor was completely replaced, 19.9 g (0.29 mol) of chlorofluoromethane and 24.6 g (0.30 mol) of trifluoroethylene were sequentially introduced.

A3. The reaction temperature was set at 10°C, the stirring rate was set at 300 rpm, and the initial reaction pressure was set at 0.9 MPa. With progressing of a reaction, the pressure was gradually decreased, and the reaction time was 10 h.

A4. After the reaction was completed, unreacted gas phase raw materials including the trifluoroethylene and/or the chlorofluoromethane and small amounts of a telomerization product and the dichloromethane were collected. The materials in the reactor were subjected to solid-liquid separation treatment such as filtration or distillation, wherein a Lewis acid catalyst (HfCl₄) was used as a solid part, and the dichloromethane and the telomerization product were used as liquid phase materials. Then, rectification and separation were carried out to obtain 3-chloro-1,1,1,2-tetrafluoropropane with a purity of 99.9% for use in the dehydrohalogenation step.

The unreacted gas phase raw materials and the separated Lewis acid catalyst were transferred back to the telomerization step for reuse.

According to analysis of the gas phase and liquid phase materials by gas chromatography, the conversion rate of chlorofluoromethane was 76.5%, the selectivity of 3-chloro-1,1,1,2-tetrafluoropropane was 81.2%, 1-chloro-1,1,2,3-tetrafluoropropane was a main by-product with a selectivity of 15.3%, and few other by-products were produced.

### 2. Dehydrohalogenation step

B1. A reaction tube made of an Inconel alloy with an inner diameter of 19 mm and a length of 800 mm was used as a fixed bed reactor. Cat 3.1 with a volume of 20 mL was filled to the middle of the fixed bed reactor, a reaction pipeline was connected, and nitrogen was introduced for purging at a flow rate of 100 mL/min.

B2. The reaction temperature was set at 350°C, and the reactor was heated at a heating rate of 5°C/min.

B3. After a catalyst bed layer reached the reaction temperature, the nitrogen flow rate was adjusted to 20 mL/min. Meanwhile, the 3-chloro-1,1,1,2-tetrafluoropropane with a purity of 99.9% was continuously introduced into the fixed bed reactor at a rate of 5.0 g/h to carry out a reaction.

B4. A gas mixture flowing out of the reactor was subjected to heat preservation treatment, followed by analysis by on-line GC and GC/MS. The conversion rate of 3-chloro-1,1,1,2-tetrafluoropropane was 88.7%, the content of 2,3,3,3-tetrafluoropropene in the product was 24.1%, and the content of 1-chloro-3,3,3-trifluoropropene was 58.7%.

### Example 3.2

The present example provides a method for co-producing 2,3,3,3-tetrafluoropropene and 1-chloro-3,3,3-trifluoropropene. The method has the same operations as that in Example 3.1, and only has the differences that in the telomerization step, 4.0 g of ZrCl₄ was used to replace the HfCl₄, the amount of chlorofluoromethane was increased to 39.7 g (0.58 mol) and the amount of trifluoroethylene was increased to 71.3 g (0.87 mol) while other conditions were remained unchanged.

According to analysis of gas phase and liquid phase materials in the telomerization step by gas chromatography, the conversion rate of chlorofluoromethane was 99.0%, the selectivity of 3-chloro-1,1,1,2-tetrafluoropropane was 89.9%, 1-chloro-1,1,2,3-tetrafluoropropane was a main by-product with a selectivity of 5.3%, and few other by-products were produced.

### Example 3.3

The present example provides a method for co-producing 2,3,3,3-tetrafluoropropene and 1-chloro-3,3,3-trifluoropropene. The method has the same operations as that in Example 3.2, and only has the differences that in the telomerization step, the dichloromethane was not used, the amount of trifluoroethylene was increased to 95.1 g (1.16 mol), meanwhile, the reaction temperature was increased to 30°C and the initial reaction pressure was increased to 1.5 MPa while other conditions were remained unchanged.

According to analysis of gas phase and liquid phase materials in the telomerization step by gas chromatography, the conversion rate of chlorofluoromethane was 99.5%, the selectivity of 3-chloro-1,1,1,2-tetrafluoropropane was 88.1%, 1-chloro-1,1,2,3-tetrafluoropropane was a main by-product with a selectivity of 4.1%, and few other by-products were produced.

### Example 3.4

The present example provides a method for co-producing 2,3,3,3-tetrafluoropropene and 1-chloro-3,3,3-trifluoropropene. The method has the same operations as that in Example 3.2, and only has the differences that in the telomerization step, 4.0 g, the same use amount, of AlCl₃ was used to replace the ZrCl₄, the dichloromethane was not used and the amount of trifluoroethylene was decreased to 52.5 g (0.64 mol) while other conditions were remained unchanged.

According to analysis of gas phase and liquid phase materials in the telomerization step by gas chromatography, the conversion rate of chlorofluoromethane was 99.6%, the selectivity of 3-chloro-1,1,1,2-tetrafluoropropane was 75.5%, 1-chloro-1,1,2,3-tetrafluoropropane was a main by-product with a selectivity of 15.9%, and few other by-products were produced.

### Example 3.5

The present example provides a method for co-producing 2,3,3,3-tetrafluoropropene and 1-chloro-3,3,3-trifluoropropene. The method has the same operations as that in Example 3.1, and only has the differences that in the step A2 of the telomerization step, after the chlorofluoromethane and the trifluoroethylene were sequentially introduced into the autoclave, high-purity and high-pressure nitrogen was used to perform pressure treatment on the autoclave so as to increase the pressure in the autoclave from 0.9 MPa to 3.0 MPa while other conditions were remained unchanged.

According to analysis of gas phase and liquid phase materials in the telomerization step by gas chromatography, the conversion rate of chlorofluoromethane was 99.8%, the selectivity of 3-chloro-1,1,1,2-tetrafluoropropane was 88.6%, 1-chloro-1,1,2,3-tetrafluoropropane was a main by-product with a selectivity of 7.6%, and few other by-products were produced.

### Example 3.6

The present example provides a method for co-producing 2,3,3,3-tetrafluoropropene and 1-chloro-3,3,3-trifluoropropene. The method has the same operations as that in Example 3.1, and only has the difference that in the dehydrohalogenation step, cat 3.2 was used to replace the cat 3.1.

According to analysis of a dehydrohalogenation reaction product by chromatography, the conversion rate of 3-chloro-1,1,1,2-tetrafluoropropane was 92.9%, the content of 2,3,3,3-tetrafluoropropene in the product was 20.3%, and the content of 1-chloro-3,3,3-trifluoropropene was 58.7%.

### Example 3.7

The present example provides a method for co-producing 2,3,3,3-tetrafluoropropene and 1-chloro-3,3,3-trifluoropropene. The method has the same operations as that in Example 3.1, and only has the difference that in the dehydrohalogenation step, the amount of the cat 3.1 was increased to 40 mL.

According to analysis of a dehydrohalogenation reaction product by chromatography, the conversion rate of 3-chloro-1,1,1,2-tetrafluoropropane was greater than 95.9%, the content of 2,3,3,3-tetrafluoropropene in the product was 16.1%, and the content of 1-chloro-3,3,3-trifluoropropene was 44.6%.

### Example 3.8

The present example provides a method for co-producing 2,3,3,3-tetrafluoropropene and 1-chloro-3,3,3-trifluoropropene. The method has the same operations as that in Example 3.1, and only has the difference that in the dehydrohalogenation step, the reaction temperature was 450°C.

According to analysis of a dehydrohalogenation reaction product by chromatography, the conversion rate of 3-chloro-1,1,1,2-tetrafluoropropane was 98.3%, the content of 2,3,3,3-tetrafluoropropene in the product was 15.9%, and the content of 1-chloro-3,3,3-trifluoropropene was 60.0%.

### Comparative Example 3.1

The present comparative example provides a method for co-producing 2,3,3,3-tetrafluoropropene and 1-chloro-3,3,3-trifluoropropene. The method has the same operations as that in Example 3.1, and only has the difference that 20 g of trichloromethane was used to replace the dichloromethane while other conditions were remained unchanged.

According to analysis of materials obtained by a reaction in the telomerization step by chromatography, the conversion rate of chlorofluoromethane was 86.9%, the selectivity of 3-chloro-1,1,1,2-tetrafluoropropane was 46.1%, a large amount of dichloromethane, as a disproportionation product of the chlorofluoromethane, was produced with a selectivity of 40.3%, and few other telomerization by-products were produced.

### Comparative Example 3.2

The present comparative example provides a method for co-producing 2,3,3,3-tetrafluoropropene and 1-chloro-3,3,3-trifluoropropene. The method has the same operations as that in Example 3.1, and only has the difference that 3.0 g of ZnCl₂ was used to replace the HfCl₄ while other conditions were remained unchanged.

According to analysis of materials obtained by a reaction in the telomerization step by chromatography, the conversion rate of chlorofluoromethane was 20.8%, and a target product, 3-chloro-1,1,1,2-tetrafluoropropane, was not produced.

### Comparative Example 3.3

The present comparative example provides a method for co-producing 2,3,3,3-tetrafluoropropene and 1-chloro-3,3,3-trifluoropropene. The method has the same operations as that in Example 3.1, and only has the difference that the HfCl₄ and the dichloromethane were not added while other conditions were remained unchanged.

According to analysis of materials obtained by a reaction in the telomerization step by chromatography, the conversion rate of chlorofluoromethane was 7.6%, a target product, 3-chloro-1,1,1,2-tetrafluoropropane, was not produced, and only a small amount of dichloromethane, as a disproportionation product of the chlorofluoromethane, was produced.

### Comparative Example 3.4

The present comparative example provides a method for co-producing 2,3,3,3-tetrafluoropropene and 1-chloro-3,3,3-trifluoropropene. The method has the same operations as that in Example 3.1, and only has the difference that in the dehydrohalogenation step, coconut shell type activated carbon pretreated by drying at 120°C for 12 h was used to replace the cat 3.1 while other conditions were remained unchanged.

According to analysis of a dehydrohalogenation reaction product by chromatography, the conversion rate of 3-chloro-1,1,1,2-tetrafluoropropane was greater than 99.7%, the content of 2,3,3,3-tetrafluoropropene in the product was 99.0%, and 1-chloro-3,3,3-trifluoropropene was not produced.

### Comparative Example 3.5

The present comparative example provides a method for co-producing 2,3,3,3-tetrafluoropropene and 1-chloro-3,3,3-trifluoropropene. The method has the same operations as that in Example 3.1, and only has the difference that a Pd/AC catalyst with a Pd supporting capacity of 1 wt% was used to replace the cat 3.1 while other conditions were remained unchanged.

According to analysis of a dehydrohalogenation reaction product by chromatography, the conversion rate of 3-chloro-1,1,1,2-tetrafluoropropane was 83.5%, the content of 2,3,3,3-tetrafluoropropene in the product was 96.4%, the content of 1-chloro-2,3,3,3-tetrafluoropropene was 1.3%, and 1-chloro-3,3,3-trifluoropropene was not produced.

## Claims

1. A two-step method for preparing 2,3,3,3-tetrafluoropropene, comprising:
A1, a telomerization step: subjecting chlorofluoromethane and trifluoroethylene to a pressure telomerization reaction under the action of a telomerization catalyst to prepare 3-chloro-1,1,1,2-tetrafluoropropane, wherein the telomerization catalyst is a Lewis acid catalyst or a mixed catalyst of a Lewis acid catalyst and dichloromethane, the Lewis acid catalyst is selected from at least one halide of Al, Sb, Ti, Zr and Hf; and
A2, a dehydrochlorination step: subjecting the 3-chloro-1,1,1,2-tetrafluoropropane to dehydrochlorination under the catalytic action of activated carbon to obtain 2,3,3,3-tetrafluoropropene, the activated carbon is selected from fruit shell type activated carbon, coal type activated carbon or wood type activated carbon.

2. The two-step method for preparing 2,3,3,3-tetrafluoropropene according to claim 1, wherein the Lewis acid catalyst is selected from at least one of ZrCl₄, HfCl₄, TiCl₄, AlCl₃, AlF₃ and SbF₅.

3. The two-step method for preparing 2,3,3,3-tetrafluoropropene according to claim 1, wherein the molar ratio of the chlorofluoromethane to the trifluoroethylene is 1:0.1 to 1:10; the Lewis acid catalyst is 0.01 to 50 wt% of the mass of the chlorofluoromethane; the molar ratio of the dichloromethane to the chlorofluoromethane is 1:0.01 to 1:10; the pressure telomerization reaction is carried out at a temperature of -30°C to 100°C and a pressure of 0.5 to 5.0 MPa for 1 to 50 h.

4. The two-step method for preparing 2,3,3,3-tetrafluoropropene according to claim 1, wherein the dehydrochlorination step is carried out at a reaction temperature of 200 to 500°C, the 3-chloro-1,1,1,2-tetrafluoropropane obtained in the telomerization step is used in the dehydrochlorination step after rectification and separation.

5. A method for co-producing 2,3,3,3-tetrafluoropropene and 1-chloro-2,3,3,3-tetrafluoropropene, comprising:
A1, a telomerization step: subjecting chlorofluoromethane and trifluoroethylene to a pressure telomerization reaction under the action of a telomerization catalyst to prepare 3-chloro-1, 1,1,2-tetrafluoropropane, wherein the telomerization catalyst is a Lewis acid catalyst or a mixed catalyst of a Lewis acid catalyst and dichloromethane, the Lewis acid catalyst is selected from at least one halide of Al, Sb, Ti, Zr and Hf; and
A2, a removal step: subjecting the 3-chloro-1,1,1,2-tetrafluoropropane to a dehydrochlorination reaction and a dehydrogenation reaction simultaneously under the action of an activated carbon supported noble metal catalyst to obtain 2,3,3,3-tetrafluoropropene and 1-chloro-2,3,3,3-tetrafluoropropene, wherein the activated carbon supported noble metal catalyst is at least one of Pd/activated carbon and Pt/activated carbon, in the activated carbon supported noble metal catalyst, the Pd or the Pt has a supporting capacity of 0.1 to 5.0 wt%, and 30-90% of the 2,3,3,3-tetrafluoropropene and 10 to 50% of the 1-chloro-2,3,3,3-tetrafluoropropene are obtained in the removal step A2.

6. The method for co-producing 2,3,3,3-tetrafluoropropene and 1-chloro-2,3,3,3-tetrafluoropropene according to claim 5, wherein the Lewis acid catalyst is selected from at least one of ZrCl₄, HfCl₄, TiCl₄, AlF₃, AlCl₃ and SbF₅.

7. The method for co-producing 2,3,3,3-tetrafluoropropene and 1-chloro-2,3,3,3-tetrafluoropropene according to claim 5, wherein the molar ratio of the chlorofluoromethane to the trifluoroethylene is 1:0.1 to 1:10; the Lewis acid catalyst is 0.01 to 50 wt% of the mass of the chlorofluoromethane; the molar ratio of the dichloromethane to the chlorofluoromethane is 1:0.01 to 1:10; the pressure telomerization reaction is carried out at a temperature of -30°C to 100°C and a pressure of 0.5 to 5.0 MPa for 1 to 50 h.

8. The method for co-producing 2,3,3,3-tetrafluoropropene and 1-chloro-2,3,3,3-tetrafluoropropene according to claim 5, wherein the activated carbon supported noble metal catalyst is prepared by an impregnation method, and the impregnation method comprises the following steps:
B1, pretreatment of a carrier: drying activated carbon at 90 to 120°C for 12 h or above;
B2, impregnation in a metal salt: impregnating the pretreated activated carbon in a soluble salt solution of Pd or Pt under vacuum or atmospheric pressure conditions;
B3, drying the impregnated activated carbon at a temperature of 90 to 120°C for 12 h or above;
and
B4, reducing the dried activated carbon by a mixed gas of hydrogen and nitrogen to obtain the activated carbon supported noble metal catalyst, wherein the hydrogen has a volume ratio of 5 to 50% in the mixed gas of hydrogen and nitrogen, and the reducing is carried out at a temperature of 150 to 300°C.

9. The method for co-producing 2,3,3,3-tetrafluoropropene and 1-chloro-2,3,3,3-tetrafluoropropene according to claim 5, wherein the 3-chloro-1,1,1,2-tetrafluoropropane is vaporized and then loaded onto a catalyst bed layer by nitrogen to carry out a removal reaction, the removal reaction has a material volume space velocity of 50 to 300 h⁻¹, and the volume ratio of N₂ to the 3-chloro-1,1,1,2-tetrafluoropropane is (0.5-3.0):1; the removal step is carried out at a reaction temperature of 300 to 600°C; the 3-chloro-1,1,1,2-tetrafluoropropane obtained in the telomerization step is used in the removal step after rectification and separation.

10. A method for co-producing 2,3,3,3-tetrafluoropropene and 1-chloro-3,3,3-trifluoropropene, comprising:
A1, a telomerization step: subjecting chlorofluoromethane and trifluoroethylene to a pressure telomerization reaction under the action of a telomerization catalyst to prepare 3-chloro-1,1,1,2-tetrafluoropropane, wherein the telomerization catalyst is a Lewis acid catalyst or a mixed catalyst of a Lewis acid catalyst and dichloromethane, the Lewis acid catalyst is selected from at least one halide of Al, Sb, Ti, Zr and Hf; and
A2, a dehydrohalogenation step: subjecting the 3-chloro-1,1,1,2-tetrafluoropropane to a dehydrochlorination reaction and a dehydrofluorination reaction simultaneously under the action of a composite dehalogenation catalyst to obtain 2,3,3,3-tetrafluoropropene and 1-chloro-3,3,3-trifluoropropene; the composite dehalogenation catalyst is prepared from at least one oxide or fluoride of Al, Mg or Cr and activated carbon powder; the activated carbon powder is selected from fruit shell type activated carbon, coal type activated carbon or wood type activated carbon.

11. The method for co-producing 2,3,3,3-tetrafluoropropene and 1-chloro-3,3,3-trifluoropropene according to claim 10, wherein the Lewis acid catalyst is selected from at least one of ZrCl₄, HfCl₄, TiCl₄, AlF₃, AlCl₃ and SbF₅.

12. The method for co-producing 2,3,3,3-tetrafluoropropene and 1-chloro-3,3,3-trifluoropropene according to claim 10, wherein the molar ratio of the chlorofluoromethane to the trifluoroethylene is 1:0.1 to 1:10; the Lewis acid catalyst is 0.01 to 50 wt% of the mass of the chlorofluoromethane; the molar ratio of the dichloromethane to the chlorofluoromethane is 1:0.01 to 1:10; the pressure telomerization reaction is carried out at a temperature of -30°C to 100°C and a pressure of 0.5 to 5.0 MPa for 1 to 50 h.

13. The method for co-producing 2,3,3,3-tetrafluoropropene and 1-chloro-3,3,3-trifluoropropene according to claim 10, wherein the at least one oxide or fluoride of Al, Mg or Cr is selected from at least one of Al₂O₃, AlF₃, MgF₂ and Cr₂O₃.

14. The method for co-producing 2,3,3,3-tetrafluoropropene and 1-chloro-3,3,3-trifluoropropene according to claim 13, wherein the content of the Al₂O₃ is 1.0 to 20 wt% of the total amount of the catalyst, the content of the AlF₃ is 1.0 to 20 wt% of the total amount of the catalyst, the content of the MgF₂ is 1.0 to 20 wt% of the total amount of the catalyst, and the content of the Cr₂O₃ is 1.0 to 20 wt% of the total amount of the catalyst.

15. The method for co-producing 2,3,3,3-tetrafluoropropene and 1-chloro-3,3,3-trifluoropropene according to claim 10, wherein the 3-chloro-1,1,1,2-tetrafluoropropane is vaporized and then loaded onto a catalyst bed layer by nitrogen to carry out a dehydrohalogenation reaction, the dehydrohalogenation reaction has a material volume space velocity of 50 to 300 h⁻¹, and the volume ratio of N₂ to the 3-chloro-1,1,1,2-tetrafluoropropane is (0.5-3.0):1; the dehydrohalogenation step is carried out at a reaction temperature of 300 to 500°C; the 3-chloro-1,1,1,2-tetrafluoropropane obtained in the telomerization step is used in the dehydrohalogenation step after rectification and separation; 10 to 50% of the 2,3,3,3-tetrafluoropropene and 10-70% of the 1-chloro-3,3,3-trifluoropropene are obtained in the dehydrohalogenation step.

16. The method for co-producing 2,3,3,3-tetrafluoropropene and 1-chloro-3,3,3-trifluoropropene according to claim 10, wherein the composite dehalogenation catalyst is prepared by a co-blending method, and the co-blending method comprises the following steps:
B1, mixing: blending Al₂O₃ and/or AlF₃ and/or MgF₂ and/or Cr₂O₃ with activated carbon powder at a mass ratio of (0.01-0.25):1 and performing thorough mixing by a mechanical stirring mode or a ball milling mode;
B2, sifting: sifting the mixed material to remove an unevenly mixed part;
B3, molding: transferring the sifted material to a tablet press for compression molding; and
B4, drying a molded catalyst to prepare the composite dehalogenation catalyst.

## Patentansprüche

1. Zweistufiges Verfahren zur Herstellung von 2,3,3,3-Tetrafluorpropen, umfassend:
A1, einen Telomerisierungsschritt: Unterwerfen von Chlorfluormethan und Trifluorethylen an eine Drucktelomerisierungsreaktion unter Einwirkung eines Telomerisierungskatalysators, um 3-Chlor-1,1,1,2-tetrafluorpropan herzustellen, wobei der Telomerisierungskatalysator ein Lewis-Säure-Katalysator oder ein Mischkatalysator eines Lewis-Säure-Katalysators und von Dichlormethan ist, wobei der Lewis-Säure-Katalysator ausgewählt ist aus wenigstens einem Halogenid von Al, Sb, Ti, Zr und Hf; und
A2, einen Dehydrochlorierungsschritt: Unterwerfen des 3-Chlor-1,1,1,2-tetrafluorpropans an Dehydrochlorierung unter katalytischer Einwirkung von Aktivkohle, um 2,3,3,3-Tetrafluorpropen zu erhalten, wobei die Aktivkohle ausgewählt ist aus Aktivkohle vom Fruchtschalentyp, Aktivkohle vom Kohletyp und Aktivkohle vom Holztyp.

2. Zweistufiges Verfahren zur Herstellung von 2,3,3,3-Tetrafluorpropen nach Anspruch 1, wobei der Lewis-Säure-Katalysator ausgewählt ist aus wenigstens einem von ZrCl4, HfCl4, TiCl4, AlCl3, AlF3 und SbF5.

3. Zweistufiges Verfahren zur Herstellung von 2,3,3,3-Tetrafluorpropen nach Anspruch 1, wobei das Molverhältnis des Chlorfluormethans zu dem Trifluorethylen 1:0,1 bis 1:10 beträgt; der Lewis-Säure-Katalysator 0,01 bis 50 Gew.- % der Masse des Chlorfluormethans entspricht; das Molverhältnis des Dichlormethans zu dem Chlorfluormethan 1:0,01 bis 1:10 beträgt; die Drucktelomerisierungsreaktion bei einer Temperatur von -30 °C bis 100 °C und einem Druck von 0,5 bis 5,0 MPa für 1 bis 50 h durchgeführt wird.

4. Zweistufiges Verfahren zur Herstellung von 2,3,3,3-Tetrafluorpropen nach Anspruch 1, wobei der Dehydrochlorierungsschritt bei einer Reaktionstemperatur von 200 bis 500 °C durchgeführt wird, wobei das bei dem Telomerisierungsschritt erhaltene 3-Chlor-1,1,1,2-tetrafluorpropan nach Rektifikation und Trennung bei dem Dehydrochlorierungsschritt verwendet wird.

5. Verfahren zur gemeinsamen Herstellung von 2,3,3,3-Tetrafluorpropen und 1-Chlor-2,3,3,3-tetrafluorpropen, umfassend:
A1, einen Telomerisierungsschritt: Unterwerfen von Chlorfluormethan und Trifluorethylen an eine Drucktelomerisierungsreaktion unter Einwirkung eines Telomerisierungskatalysators, um 3-Chlor-1,1,1,2-tetrafluorpropan herzustellen, wobei der Telomerisierungskatalysator ein Lewis-Säure-Katalysator oder ein Mischkatalysator eines Lewis-Säure-Katalysators und von Dichlormethan ist, wobei der Lewis-Säure-Katalysator ausgewählt ist aus wenigstens einem Halogenid von Al, Sb, Ti, Zr und Hf; und
A2, einen Entfernungsschritt: Unterwerfen des 3-Chlor-1,1,1,2-tetrafluorpropans an eine Dehydrochlorierungsreaktion und gleichzeitig eine Dehydrierungsreaktion unter der Wirkung eines Aktivkohle-getragenen Edelmetallkatalysators, um 2,3,3,3-Tetrafluorpropen und 1-Chlor-2,3,3,3-tetrafluorpropen zu erhalten, wobei der Aktivkohle-getragene Edelmetallkatalysator wenigstens einer von Pd/Aktivkohle und Pt/Aktivkohle ist, wobei bei dem Aktivkohle-getragenen Edelmetallkatalysator das Pd oder Pt eine getragene Kapazität von 0,1 bis 5,0 Gew.-% aufweist, und 30-90 % des 2,3,3,3-Tetrafluorpropens und 10 bis 50 % des 1-Chlor-2,3,3,3-tetrafluorpropens bei dem Entfernungsschritt A2 erhalten werden.

6. Verfahren zur gemeinsamen Herstellung von 2,3,3,3-Tetrafluorpropen und 1-Chlor-2,3,3,3-tetrafluorpropen nach Anspruch 5, wobei der Lewis-Säure-Katalysator ausgewählt ist aus wenigstens einem von ZrCl4, HfCl4, TiCl4, AlF3, AlCl3 und SbF5.

7. Verfahren zur gemeinsamen Herstellung von 2,3,3,3-Tetrafluorpropen und 1-Chlor-2,3,3,3-tetrafluorpropen nach Anspruch 5, wobei das Molverhältnis des Chlorfluormethans zu dem Trifluorethylen 1:0,1 bis 1:10 beträgt; der Lewis-Säure-Katalysator 0,01 bis 50 Gew.- % der Masse des Chlorfluormethans entspricht; das Molverhältnis des Dichlormethans zu dem Chlorfluormethan 1:0,01 bis 1:10 beträgt; die Drucktelomerisierungsreaktion bei einer Temperatur von -30 °C bis 100 °C und einem Druck von 0,5 bis 5,0 MPa für 1 bis 50 h durchgeführt wird.

8. Verfahren zur gemeinsamen Herstellung von 2,3,3,3-Tetrafluorpropen und 1-Chlor-2,3,3,3-tetrafluorpropen nach Anspruch 5, wobei der Aktivkohle-getragene Edelmetallkatalysator durch ein Imprägnierverfahren hergestellt wird und das Imprägnierverfahren die folgenden Schritte umfasst:
B1, Vorbehandlung eines Trägers: Trocknen von Aktivkohle bei 90 bis 120 °C für 12 h oder mehr;
B2, Imprägnierung in einem Metallsalz: Imprägnieren der vorbehandelten Aktivkohle in einer löslichen Salzlösung von Pd oder Pt unter Vakuum- oder Atmosphärendruckbedingungen;
B3, Trocknen der imprägnierten Aktivkohle bei einer Temperatur von 90 bis 120 °C für 12 h oder mehr; und
B4, Reduzieren der getrockneten Aktivkohle durch ein Mischgas aus Wasserstoff und Stickstoff, um den Aktivkohle-getragenen Edelmetallkatalysator zu erhalten, wobei der Wasserstoff in dem Mischgas aus Wasserstoff und Stickstoff einen Volumenanteil von 5 bis 50 % aufweist und die Reduktion bei einer Temperatur von 150 bis 300 °C durchgeführt wird.

9. Verfahren zur gemeinsamen Herstellung von 2,3,3,3-Tetrafluorpropen und 1-Chlor-2,3,3,3-tetrafluorpropen nach Anspruch 5, wobei das 3-Chlor-1,1,1,2-tetrafluorpropan verdampft und anschließend zur Durchführung einer Entfernungsreaktion durch Stickstoff auf eine Katalysatorbettschicht geladen wird, die Entfernungsreaktion eine Material-Volumen-Raumgeschwindigkeit von 50 bis 300 h-1 aufweist und das Volumenverhältnis von N2 zu dem 3-Chlor-1,1,1,2-tetrafluorpropan (0,5-3,0):1 beträgt; der Entfernungsschritt bei einer Reaktionstemperatur von 300 bis 600 °C durchgeführt wird; das bei dem Telomerisierungsschritt erhaltene 3-Chlor-1,1,1,2-tetrafluorpropan nach Rektifikation und Abtrennung bei dem Entfernungsschritt verwendet wird.

10. Verfahren zur gemeinsamen Herstellung von 2,3,3,3-Tetrafluorpropen und 1-Chlor-3,3,3-trifluorpropen, umfassend:
A1, einen Telomerisierungsschritt: Unterwerfen von Chlorfluormethan und Trifluorethylen an eine Drucktelomerisierungsreaktion unter Einwirkung eines Telomerisierungskatalysators, um 3-Chlor-1,1,1,2-tetrafluorpropan herzustellen, wobei der Telomerisierungskatalysator ein Lewis-Säure-Katalysator oder ein Mischkatalysator eines Lewis-Säure-Katalysators und von Dichlormethan ist, wobei der Lewis-Säure-Katalysator ausgewählt ist aus wenigstens einem Halogenid von Al, Sb, Ti, Zr und Hf; und
A2, ein Dehydrohalogenierungsschritt: Unterwerfen des 3-Chlor-1,1,1,2-tetrafluorpropans an eine Dehydrochlorierungsreaktion und gleichzeitig eine Dehydrofluorierungsreaktion unter der Wirkung eines Dehyhalogenierungs-Verbundkatalysators, um 2,3,3,3-Tetrafluorpropen und 1-Chlor-3,3,3-trifluorpropen zu erhalten; wobei der Dehyhalogenierungs-Verbundkatalysator aus wenigstens einem Oxid oder Fluorid von Al, Mg oder Cr und Aktivkohlepulver hergestellt ist; wobei das Aktivkohlepulver ausgewählt ist aus Aktivkohle vom Fruchtschalentyp, Aktivkohle vom Kohletyp und Aktivkohle vom Holztyp.

11. Verfahren zur gemeinsamen Herstellung von 2,3,3,3-Tetrafluorpropen und 1-Chlor-3,3,3-trifluorpropen nach Anspruch 10, wobei der Lewis-Säure-Katalysator ausgewählt ist aus wenigstens einem von ZrCl4, HfCl4, TiCl4, AlF3, AlCl3 und SbF5.

12. Verfahren zur gemeinsamen Herstellung von 2,3,3,3-Tetrafluorpropen und 1-Chlor-3,3,3-Trifluorpropen nach Anspruch 10, wobei das Molverhältnis des Chlorfluormethans zu dem Trifluorethylen 1:0,1 bis 1:10 beträgt; der Lewis-Säure-Katalysator 0,01 bis 50 Gew.- % der Masse des Chlorfluormethans entspricht; das Molverhältnis des Dichlormethans zu dem Chlorfluormethan 1:0,01 bis 1:10 beträgt; die Drucktelomerisierungsreaktion bei einer Temperatur von -30 °C bis 100 °C und einem Druck von 0,5 bis 5,0 MPa für 1 bis 50 h durchgeführt wird.

13. Verfahren zur gemeinsamen Herstellung von 2,3,3,3-Tetrafluorpropen und 1-Chlor-3,3,3-trifluorpropen nach Anspruch 10, wobei das wenigstens eine Oxid oder Fluorid von Al, Mg oder Cr ausgewählt ist aus wenigstens einem von Al2O3, AlF3, MgF2 und Cr2O3.

14. Verfahren zur gemeinsamen Herstellung von 2,3,3,3-Tetrafluorpropen und 1-Chlor-3,3,3-trifluorpropen nach Anspruch 13, wobei der Gehalt an dem Al2O3 1,0 bis 20 Gew.-% der Gesamtmenge des Katalysators beträgt, der Gehalt an dem AlF3 1,0 bis 20 Gew.-% der Gesamtmenge des Katalysators beträgt, der Gehalt an dem MgF2 1,0 bis 20 Gew.-% der Gesamtmenge des Katalysators beträgt und der Gehalt an dem Cr2O3 1,0 bis 20 Gew.-% der Gesamtmenge des Katalysators beträgt.

15. Verfahren zur gemeinsamen Herstellung von 2,3,3,3-Tetrafluorpropen und 1-Chlor-3,3,3-trifluorpropen nach Anspruch 10, wobei das 3-Chlor-1,1,1,2-tetrafluorpropan verdampft und dann durch Stickstoff auf eine Katalysatorbettschicht geladen wird, um eine Dehydrohalogenierungsreaktion durchzuführen, die Dehydrohalogenierungsreaktion eine Material-Volumen-Raumgeschwindigkeit von 50 bis 300 h-1 aufweist und das Volumenverhältnis von N2 zu dem 3-Chlor-1,1,1,2-tetrafluorpropan (0,5-3,0):1 beträgt; der Dehydrohalogenierungsschritt bei einer Reaktionstemperatur von 300 bis 500 °C durchgeführt wird; das bei dem Telomerisierungsschritt erhaltene 3-Chlor-1,1,1,2-tetrafluorpropan nach Rektifikation und Abtrennung bei dem Dehydrohalogenierungsschritt verwendet wird; wobei 10 bis 50 % des 2,3,3,3-Tetrafluorpropens und 10-70 % des 1-Chlor-3,3,3-trifluorpropens bei dem Dehydrohalogenierungsschritt erhalten werden.

16. Verfahren zur gemeinsamen Herstellung von 2,3,3,3-Tetrafluorpropen und 1-Chlor-3,3,3-trifluorpropen nach Anspruch 10, wobei der Dehalogenierungs-Verbundkatalysator durch ein Co-Misch-Verfahren hergestellt wird und das Co-Misch-Verfahren die folgenden Schritte umfasst:
B1, Mischen: Vermischen von Al2O3 und/oder AlF3 und/oder MgF2 und/oder Cr2O3 mit Aktivkohlepulver in einem Masseverhältnis von (0,01-0,25): 1 und Durchführen von gründlichem Mischen durch einen mechanischen Rührmodus oder einen Kugelmahlmodus;
B2, Sieben: Sieben des gemischten Materials, um einen ungleichmäßig gemischten Teil zu entfernen;
B3, Formen: Überführen des gesiebten Materials zu einer Tablettenpresse zum Formpressen; und
B4, Trocknen eines geformten Katalysators, um den Dehalogenierungs-Verbundkatalysator herzustellen.

## Revendications

1. Procédé en deux étapes de préparation de 2,3,3,3-tétrafluoropropène comprenant :
A1, une étape de télomérisation : soumission de chlorofluorométhane et de trifluoroéthylène à une réaction de télomérisation sous pression sous l'action d'un catalyseur de télomérisation pour préparer du 3-chloro-1,1,1,2-tétrafluoropropane, dans lequel le catalyseur de télomérisation est un catalyseur d'acide de Lewis ou un catalyseur mixte d'un catalyseur d'acide de Lewis et de dichlorométhane, le catalyseur d'acide de Lewis est choisi parmi au moins un halogénure de Al, Sb, Ti, Zr et Hf ; et
A2, une étape de déshydrochloration : soumission du 3-chloro-1,1,1,2-tétrafluoropropane à une déshydrochloration sous l'action catalytique de carbone activé pour obtenir le 2,3,3,3-tétrafluoropropène, le carbone activé est sélectionné parmi un carbone activé de type coque de fruit, un carbone activé de type charbon ou un carbone activé de type bois.

2. Procédé en deux étapes de préparation de 2,3,3,3-tétrafluoropropène selon la revendication 1, dans lequel le catalyseur d'acide de Lewis est choisi parmi au moins l'un parmi ZrCl₄, HfCl₄, TiCl₄, AlCl₃, AlF₃ et SbF₅.

3. Procédé en deux étapes de préparation de 2,3,3,3-tétrafluoropropène selon la revendication 1, dans lequel le rapport molaire du chlorofluorométhane sur le trifluoroéthylène est de 1:0,1 à 1:10 ; le catalyseur d'acide de Lewis est 0,01 à 50 % en poids de la masse du chlorofluorométhane ; le rapport molaire du dichlorométhane sur le chlorofluorométhane est de 1:0,01 à 1:10 ; la réaction de télomérisation sous pression est effectuée à une température de -30 °C à 100 °C et une pression de 0,5 à 5,0 MPa pendant 1 à 50 h.

4. Procédé en deux étapes de préparation de 2,3,3,3-tétrafluoropropène selon la revendication 1, dans lequel l'étape de déshydrochloration est effectuée à une température de réaction de 200 à 500 °C, le 3-chloro-1,1,1,2-tétrafluoropropane obtenu dans l'étape de télomérisation est utilisé dans l'étape de déshydrochloration après rectification et séparation.

5. Procédé de coproduction de 2,3,3,3-tétrafluoropropène et de 1-chloro-2,3,3,3-tetrafluoropropène comprenant :
A1, une étape de télomérisation : soumission de chlorofluorométhane et de trifluoroéthylène à une réaction de télomérisation sous pression sous l'action d'un catalyseur de télomérisation pour préparer du 3-chloro-1,1,1,2-tétrafluoropropane, dans lequel le catalyseur de télomérisation est un catalyseur d'acide de Lewis ou un catalyseur mixte d'un catalyseur d'acide de Lewis et de dichlorométhane, le catalyseur d'acide de Lewis est choisi parmi au moins un halogénure de Al, Sb, Ti, Zr et Hf ; et
A2, une étape d'élimination : soumission du 3-chloro-1,1,1,2-tétrafluoropropane à une réaction de déshydrochloration et une réaction de déshydrogénation simultanément sous l'action d'un catalyseur de métal noble supporté sur carbone activé pour obtenir du 2,3,3,3-tétrafluoropropène et du 1-chloro-2,3,3,3-tétrafluoropropène, dans lequel le catalyseur de métal noble supporté sur carbone activé est au moins l'un parmi Pd/carbone activé et Pt/carbone activé, dans le catalyseur de métal noble supporté sur carbone activé, le Pd ou le Pt possède une capacité support de 0,1 à 5,0 % en poids, et 30-90 % du 2,3,3,3-tétrafluoropropène et 10 à 50 % du 1-chloro-2,3,3,3-tetrafluoropropène sont obtenus dans l'étape d'élimination A2.

6. Procédé de coproduction de 2,3,3,3-tétrafluoropropène et de 1-chloro-2,3,3,3-tétrafluoropropène selon la revendication 5, dans lequel le catalyseur d'acide de Lewis est choisi parmi au moins l'un parmi ZrCl4, HfCl4, TiCl4, AlF3, AlCl3 et SbF5.

7. Procédé de coproduction de 2,3,3,3-tétrafluoropropène et de 1-chloro-2,3,3,3-tétrafluoropropène selon la revendication 5, dans lequel le rapport molaire du chlorofluorométhane sur le trifluoroéthylène est de 1:0,1 à 1:10 ; le catalyseur d'acide de Lewis est 0,01 à 50 % en poids de la masse du chlorofluorométhane ; le rapport molaire du dichlorométhane sur le chlorofluorométhane est de 1:0,01 à 1:10 ; la réaction de télomérisation sous pression est effectuée à une température de -30 °C à 100 °C et une pression de 0,5 à 5,0 MPa pendant 1 à 50 h.

8. Procédé de coproduction de 2,3,3,3-tétrafluoropropène et de 1-chloro-2,3,3,3-tétrafluoropropène selon la revendication 5, dans lequel le catalyseur de métal noble supporté sur carbone activé est préparé par un procédé d'imprégnation, et le procédé d'imprégnation comprend les étapes suivantes :
B1, prétraitement d'un support : séchage de carbone activé à 90 à 120 °C pendant 12 h ou au-delà ;
B2, imprégnation dans un sel métallique : imprégnation du carbone activé prétraité dans une solution de sel soluble de Pd ou Pt dans des conditions de vide ou de pression atmosphérique ;
B3, séchage du carbone activé imprégné à une température de 90 à 120 °C pendant 12 h ou au-delà ; et
B4, réduction du carbone activé séché par un gaz mixte d'hydrogène et d'azote pour obtenir le catalyseur de métal noble supporté sur carbone activé, dans lequel l'hydrogène a un rapport volumique de 5 à 50 % dans le gaz mixte d'hydrogène et d'azote, et la réduction est effectuée à une température de 150 à 300 °C.

9. Procédé de coproduction de 2,3,3,3-tétrafluoropropène et de 1-chloro-2,3,3,3-tétrafluoropropène selon la revendication 5, dans lequel le 3-chloro-1,1,1,2-tétrafluoropropane est vaporisé et ensuite chargé sur une couche de lit de catalyseur par de l'azote pour effectuer une réaction d'élimination, la réaction d'élimination a une vitesse volumique de matières de 50 à 300 h-1, et le rapport volumique de N2 sur 3-chloro-1,1,1,2-tétrafluoropropane est (0,5-3,0):1 ; l'étape d'élimination est effectuée à une température de réaction de 300 à 600 °C ; le 3-chloro-1,1,1,2-tétrafluoropropane obtenu dans l'étape de télomérisation est utilisé dans l'étape d'élimination après rectification et séparation.

10. Procédé de coproduction de 2,3,3,3-tétrafluoropropène et de 1-chloro-3,3,3-trifluoropropène comprenant :
A1, une étape de télomérisation : soumission de chlorofluorométhane et de trifluoroéthylène à une réaction de télomérisation sous pression sous l'action d'un catalyseur de télomérisation pour préparer du 3-chloro-1,1,1,2-tétrafluoropropane, dans lequel le catalyseur de télomérisation est un catalyseur d'acide de Lewis ou un catalyseur mixte d'un catalyseur d'acide de Lewis et de dichlorométhane, le catalyseur d'acide de Lewis est choisi parmi au moins un halogénure de Al, Sb, Ti, Zr et Hf ; et
A2, une étape de déshydrohalogénation : soumission du 3-chloro-1,1,1,2-tétrafluoropropane à une réaction de déshydrochloration et une réaction de déshydrofluoration simultanément sous l'action d'un catalyseur de déshydrohalogénation composite pour obtenir du 2,3,3,3-tétrafluoropropène et du 1-chloro-3,3,3-trifluoropropène ; le catalyseur de déshydrohalogénation composite est préparé à partir d'au moins un oxyde ou fluorure de Al, Mg ou Cr et poudre de carbone activé ; la poudre de carbone activé est choisie parmi du carbone activé de type coque de fruit, du carbone activé de type charbon ou du carbone activé de type bois.

11. Procédé de coproduction de 2,3,3,3-tétrafluoropropène et de 1-chloro-3,3,3-trifluoropropène selon la revendication 10, dans lequel le catalyseur d'acide de Lewis est choisi parmi au moins l'un parmi ZrCl4, HfCl4, TiCl4, AlF3, AlCl3 et SbF5.

12. Procédé de coproduction de 2,3,3,3-tétrafluoropropène et de 1-chloro-3,3,3-trifluoropropène selon la revendication 10, dans lequel le rapport molaire du chlorofluorométhane sur le trifluoroéthylène est de 1:0,1 à 1:10 ; le catalyseur d'acide de Lewis est 0,01 à 50 % en poids de la masse du chlorofluorométhane ; le rapport molaire du dichlorométhane sur le chlorofluorométhane est de 1:0,01 à 1:10 ; la réaction de télomérisation sous pression est effectuée à une température de -30 °C à 100 °C et une pression de 0,5 à 5,0 MPa pendant 1 à 50 h.

13. Procédé de coproduction de 2,3,3,3-tétrafluoropropène et de 1-chloro-3,3,3-trifluoropropène selon la revendication 10, dans lequel l'au moins un oxyde ou fluorure de Al, Mg ou Cr est choisi parmi au moins l'un parmi Al2O3, AlF3, MgF2 et Cr2O3.

14. Procédé de coproduction de 2,3,3,3-tétrafluoropropène et de 1-chloro-3,3,3-trifluoropropène selon la revendication 13, dans lequel la teneur en Al2O3 est de 1,0 à 20 % en poids de la quantité totale du catalyseur, la teneur en AlF3 est de 1,0 à 20 % en poids de la quantité totale du catalyseur, la teneur en MgF2 est de 1,0 à 20 % en poids de la quantité totale du catalyseur, et la teneur en Cr2O3 est de 1,0 à 20 % en poids de la quantité totale du catalyseur.

15. Procédé de coproduction de 2,3,3,3-tétrafluoropropène et de 1-chloro-3,3,3-trifluoropropène selon la revendication 10, dans lequel le 3-chloro-1,1,1,2-tétrafluoropropane est vaporisé et ensuite chargé sur une couche de lit de catalyseur par de l'azote pour effectuer une réaction de déshydrohalogénation, la réaction de déshydrohalogénation a une vitesse volumique de matières de 50 à 300 h-1, et le rapport volumique de N2 sur le 3-chloro-1,1,1,2-tétrafluoropropane est (0,5-3,0):1 ; le 3-chloro-1,1,1,2-tétrafluoropropane obtenu dans l'étape de télomérisation est utilisé dans l'étape de déshydrohalogénation après rectification et séparation ; 10 à 50 % de 2,3,3,3-tétrafluoropropène et 10-70 % de 1-chloro-3,3,3-trifluoropropène sont obtenus dans l'étape de déshydrohalogénation.

16. Procédé de coproduction de 2,3,3,3-tétrafluoropropène et de 1-chloro-3,3,3-trifluoropropène selon la revendication 10, dans lequel le catalyseur de déshydrohalogénation composite est préparé par le procédé de co-mélange, et le procédé de co-mélange comprend les étapes suivantes :
B1, mélange : mélange de Al2O3 et/ou AlF3 et/ou MgF2 et/ou Cr2O3 avec de la poudre de carbone activé à un rapport massique de (0,01-0,25): 1 et réalisation d'un mélange intime par un mode d'agitation mécanique ou un mode de broyage à billes ;
B2, tamisage : criblage du matériau mélangé pour enlever une partie mélangée de manière non uniforme ;
B3, moulage : transfert du matériau criblé à une presse à comprimés pour moulage par compression ;
et
B4, séchage d'un catalyseur moulé pour préparer le catalyseur de déshydrohalogénation composite.
